# EUROPÄISCHE PATENTSCHRIFT

(11) **EP 3 763 335 B1**
(45) Veröffentlichungstag und Bekanntmachung des Hinweises auf die Patenterteilung: **03.07.2024**
(21) Anmeldenummer: 19185710.1
(22) Anmeldetag: 11.07.2019
(51) Int. Cl.: A61F 2/32, A61F 2/36, A61F 2/38, A61F 2/30, A61F 2/46

(54) **GELENKIMPLANTAT FÜR EINE VERABREICHUNG EINES ARZNEIMITTELS**
JOINT IMPLANT FOR ADMINISTRATION OF A MEDICAMENT
IMPLANT ARTICULAIRE POUR UNE ADMINISTRATION D'UN MÉDICAMENT

(43) Veröffentlichungstag der Anmeldung: 13.01.2021
(73) Patentinhaber: Waldemar Link GmbH & Co. KG, 22339 Hamburg (DE)
(72) Erfinder: LINK, Helmut D., 22397 Hamburg (DE); FISCHER, Hans-Joachim, 22850 Norderstedt (DE)
(74) Vertreter: AWA Denmark A/S

(56) Entgegenhaltungen:
- EP-A1- 3 216 425
- EP-A1- 3 542 759
- US-A- 4 219 893
- US-A1- 2010 217 401
- US-A1- 2014 194 811
- US-A1- 2016 367 371

## Beschreibung

### GEBIET DER ERFINDUNG

Die Erfindung betrifft eine Gelenkimplantatkomponente für eine Verabreichung eines Arzneimittels. Zudem betrifft die Erfindung ein verschwenkbares Gelenkimplantat zur Verabreichung eines Arzneimittels, das eine erste und eine zweite Gelenkimplantatkomponente für eine Verabreichung eines Arzneimittels aufweist.

### STAND DER TECHNIK

Gelenkimplantate haben sich als Ersatz für nicht mehr funktionstüchtige Gelenke bewährt und sind ein alltäglicher Eingriff. Allerdings bedingt die Größe von Gelenkimplantaten, dass ihre Implantation nach wie vor ein vergleichsweise gro-ßer Eingriff ist. Bei einem solchen Eingriff wird ein Implantat in den Körper eines Patienten eingebracht, das eine relativ große Fläche aufweist. Diese Implantatfläche ist vor der Implantation der Umgebung ausgesetzt, sodass es schwierig ist, am Eingriffsort absolute Sterilität sicherzustellen.

Folglich kann es vorkommen, dass im Bereich eines behandelten Gelenkbereichs eine Infektion auftritt. Neben oben genannten hygienischen Risikofaktoren gibt es zudem auch patientenspezifische Risikofaktoren, die eine solche Infektion hervorrufen können. Auch wenn eine Infektion relativ selten vorkommt, bedeutet ihr Auftreten für den einzelnen betroffenen Patienten eine hohe Belastung.

Infektionen der genannten Art werden im Allgemeinen durch Verabreichung von Wirkstoffen, wie zum Beispiel Antibiotika, bekämpft. Gerade bei Risikopatienten kann eine solche Behandlung auch vorbeugend durchgeführt werden. Die Wirkstoffe werden dabei direkt am Implantationsort freigesetzt. Hierfür kommen zum Beispiel Kollagenschwämme, Vliese oder Ketten mit Knochenzementkügelchen zum Einsatz, in denen sich ein Wirkstoff befindet, der nach und nach freigegeben wird, um Entzündungen zu bekämpfen oder gegen diese vorzubeugen. Bringt die Behandlung nicht den gewünschten Erfolg, kann eine Revision des zuvor implantierten Gelenkimplantat notwendig sein. Ähnliches gilt auch im Falle einer Gewebewucherung, wie z. B. einem Tumor.

Bei der Revision wird das im Körper befindliche Gelenkimplantat wieder entnommen, um den entzündeten Bereich besser behandeln zu können. Als Folge der Explantation fehlt ein Teil des Stützapparats, was die Mobilität des Patienten erheblich einschränkt und zudem zu einer Verkürzung des umliegenden Weichteilgewebes führt. Eine direkte Neuimplantation, um einen solchen Effekt zu verhindern, ist jedoch nicht möglich, da die Infektion zuvor vollständig abklingen muss.

Um der Verkürzung des Weichteilgewebes vorzubeugen, wurde deswegen ein Verfahren entwickelt, bei dem der durch die Explantation entstandene Leerraum teilweise durch Knochenzement aufgefüllt wird. Hierzu wird der im Operationssaal angerührte Knochenzement im noch plastischen Zustand an den auszufüllenden Hohlraum angepasst. Um eine optimale Anpassung eines solchen Knochenzementspacers zu gewährleisten, wird der Knochenzement unter Berücksichtigung des verbleibenden Knochengewebes des Patienten modelliert. Da der Knochenzement im plastischen Zustand allerdings noch nicht ausgehärtet ist und somit in ihm weiterhin eine endotherme Aushärtungsreaktion stattfindet, besteht das Risiko durch zu hohe Temperaturen das umliegende Gewebe bis hin zu einer Nekrose zu schädigen. Wenn über den Knochenzement eine Wirkstoffabgabe erfolgen soll, kann die Wärme zudem auch negative Auswirkungen auf den Wirkstoff zeigen.

Trotz einer solchen Verwendung eines Knochenzementspacers muss der Patient weiterhin ruhiggestellt werden, da dessen Materialeigenschaften und die mechanische Anbindung an das verbleibende Knochengewebe trotz Anmodellierens unzureichend sind. Es handelt sich bei dem Knochenzement also im Wesentlichen um ein Material, welches den Raum des entnommenen Gelenkimplantats und sezierten Knochens einnimmt, jedoch mechanisch nicht in der Lage ist, eine Mobilisierung des Patienten zu ermöglichen. Im Ergebnis kann ein Knochenzementspacer also weder die Stützfunktion noch die Beweglichkeit des explantierten Gelenkersatzes ersetzen. Daraus folgt, dass der durch die Immobilisation bedingten Weichteilkontraktur, Muskelatrophie und Knochenschwächung auch bei Verwendung eines Spacers nur bedingt entgegengewirkt werden kann.

Es wurde deswegen vorgeschlagen, nach der Revision des Gelenkersatzes ein Platzhalterimplantat einzusetzen, das einen über Infusionsschläuche zugeführten Wirkstoff zu dem betroffenen Gelenkbereich führt und dort verabreicht. So offenbart die US 9,707,008 B2 ein Platzhalterimplantat für den Kniegelenkbereich, das auf Höhe des vormaligen Kniegelenks ein hohles Kupplungsstück aufweist, an dem an gegenüberliegenden Seiten hohle intramedulläre Stäbe für die Tibia und das Femur angebracht sind. Ein dem Kupplungsstück zugeführter Wirkstoff kann durch entsprechend angeordnete Öffnungen über das Kupplungsstück in die Stäbe geführt und an deren distalen Enden in das umliegende Gewebe abgegeben werden. Die intramedullären Stäbe können zudem an ihrer Außenseite Längsnuten aufweisen, um den Wirkstoff außen an dem jeweiligen intramedullären Stab durch Öffnungen in dem Kupplungsstück in dessen Hohlraum zurückzuführen und so den Wirkstoff umzuwälzen.

Auch wenn der Bänderapparat durch das Platzhalterimplantat der US 9,707,008 B2 unter Spannung gehalten wird, bedeutet dies für den Patienten keinerlei Mobilität, da die intramedullären Stäbe des Platzhalterimplantats lediglich in den Knochen eingeführt werden. Zudem ist das Zuführen und Umwälzen des Wirkstoffs davon abhängig, dass die äußeren Längsnuten nach Einsetzen eines intramedullären Stabs freigehalten werden können. Weiterhin besteht die Gefahr, dass infektiöses Material im Gelenkbereich umgewälzt wird und so unabsichtlich verteilt wird.

Auch in EP 3542759 A1 wird ein Platzhalterimplantat mit Spülfunktion offenbart. Mit diesem lassen sich zwei unabhängige Spülkreisläufe, unter anderem entlang des Implantatschafts, realisieren. EP 3542759 A1 offenbart die technischen Merkmale des Präambelteils des Anspruchs 1.

### ZUSAMMENFASSUNG DER ERFINDUNG

Folglich war es eine Aufgabe, nach Revision eines Gelenkersatzes die Versorgung eines entzündeten Gewebebereichs mit einem Wirkstoff zu verbessern. Eine weitere Aufgabe war es, einer Ausweitung des entzündeten Bereichs entgegenzuwirken. Zudem sollte dem Patienten nach Entnahme des von der Infektion betroffenen Gelenkersatzes durch einen entsprechenden Ersatz ein gewisses Ausmaß an Mobilität ermöglicht werden.

Angesichts dieser Aufgaben wird eine Gelenkimplantatkomponente nach Anspruch 1 für eine Verabreichung eines Arzneimittels bereitgestellt, die einen Implantatschaft, einen am proximalen Ende des Implantatschafts angeordneten Gelenkabschnitt, eine Spülzuführöffnung und eine Rückspülöffnung aufweist. Vorteilhafte Ausführungsformen der Erfindung sind in den Unteransprüchen aufgeführt. Innerhalb des Implantatschafts erstrecken sich mindestens ein Schaftspülkanal und ein Schaftrückspülkanal entlang des Implantatschafts. Der Schaftspülkanal ist für eine Abgabe des Arzneimittels über Schaftspüllöcher und der Schaftrückspülkanal für eine Aufnahme des abgegebenen Arzneimittels über mindestens ein Schaftrückspülloch mit der Außenseite des Implantatschafts verbunden. Die Spülzuführöffnung steht mit dem proximalen Ende des Schaftspülkanals in Fluidverbindung. Die Rückspülöffnung steht mit dem proximalen Ende des Schaftrückspülkanals in Fluidverbindung.

Eine derartige Gelenkimplantatkomponente stellt ein Interimsimplantat dar, welches zur Behandlung eines entzündeten Gewebebereichs zwischenzeitlich als Ersatz für ein vollwertiges Gelenkimplantat implantiert werden kann. Während der Behandlungszeit ermöglicht diese Gelenkimplantatkomponente neben der Behandlung dem Patienten oder der Patientin ein eingeschränktes Maß an Mobilität. Letzteres beugt insbesondere gegen eine durch mangelnde Bewegung verursachte Muskelschwäche und gegen eine übermäßige Verkürzung des Bänderapparats vor. Eine solche Verkürzung des Bänderapparats kann ansonsten ohne Ersatz für die entzündete vollwertige Gelenkimplantatkomponente aufgrund des durch die Explantation entstehenden Hohlraums auftreten.

Um mit Hilfe der Implantatkomponente den Patienten besser mobilisieren zu können, ist die Gelenkimplantatkomponente vorzugsweise über Knochenzement möglichst knochenschonend im Bereich der Zementkontaktflächen fixierbar.

Es ist möglich, die Gelenkimplantatkomponente so auszuführen, dass sie mit einer vollwertigen gegenüberliegenden korrespondierenden Gelenkimplantatkomponente ähnlich wie die zuvor explantierte vollwertige Gelenkimplantatkomponente zusammenwirkt. Mit anderen Worten kann die Gelenkimplantatkomponente die von einer Infektion betroffene Seite eines vollwertigen Gelenkimplantats ersetzen, sodass der Gelenkabschnitt der Gelenkimplantatkomponente mit einem Gelenkabschnitt der gegenüberliegenden vollwertigen Gelenkimplantatkomponente zusammenwirkt.

Unter einer vollwertigen Gelenkimplantatkomponente ist dabei eine Gelenkimplantatkomponente zu verstehen, welche ausgelegt ist, die Aufgabe eines nativen Gelenks langfristig zu übernehmen und die folglich nicht mit einer Arzneimittelversorgung ausgeführt ist.

Der Schaftspülkanal und die Schaftspüllöcher ermöglichen eine gezielte Verabreichung eines Wirkstoffes, insbesondere eines entzündungshemmenden Wirkstoffes, wie zum Beispiel ein Antibiotikum. Auch können über das Schaftrückspülloch und den Schaftrückspülkanal Flüssigkeit und andere Rückstände aus dem entzündeten Bereich entfernt werden, d. h. es kann eine Drainage des entzündeten Bereichs ausgeführt werden. Auf die Flüssigkeit und andere Rückstände wird nachfolgend einfach als Drainageflüssigkeit Bezug genommen. Eine solche Drainage trägt zumindest dazu bei, den entzündeten Bereich zu reinigen und damit zu einer schnelleren Bekämpfung der Infektion. Zudem beugt die Drainage einer weiteren Ausbreitung der Infektion vor.

Sowohl bei dem Schaftspülkanal als auch bei dem Schaftrückspülkanal handelt es sich um geschlossene bzw. rohrartige Kanäle. Dementsprechend erfolgt eine Abgabe des Arzneimittels im Falle des Implantats (ausschließlich) über die Schaftspüllöcher. Gleichermaßen erfolgt die Aufnahme der Drainageflüssigkeit im Schaftbereich des Implantats (ausschließlich) über das mindestens eine Schaftrückspülloch. Damit erfolgt sowohl die Abgabe des Arzneimittels als auch die Aufnahme der Drainageflüssigkeit gezielter und kontrollierter.

Dadurch, dass die Schaftspüllöcher und das mindestens eine Schaftrückspülloch im Schaftbereich der Gelenkimplantatkomponente vorgesehen sind, der sich im implantierten Zustand in der Explantationsaussparung des Knochengewebes befindet, lässt sich eine Infektion lokal gezielt behandeln.

Bei einer Ausführungsform weisen der Implantatschaft und der Gelenkabschnitt jeweils einen vorzugsweise mit einem Gewinde ausgebildeten Verbindungsabschnitt auf, über den sie miteinander montierbar sind.

Bei dieser Ausführungsform ist es dementsprechend möglich, die Gelenkimplantatkomponente modular an die anatomische Situation eines Patienten anzupassen. Insbesondere kann der Implantatschaft mit den Schaftspüllöchern an die im Knochengewebe vorhandene Explantationsaussparung angepasst werden. Die Auswahl des Implantatschafts erfolgt dabei so, dass zwischen der Außenseite des Schafts und dem Knochengewebe ein Spalt vorhanden ist. In diesen Spalt kann dann das Arzneimittel dem entzündeten Bereich zugeführt werden. Gleichermaßen wird der Gelenkabschnitt gegebenenfalls so ausgewählt, dass er mit einem möglicherweise vorhandenen und oben bereits erwähnten korrespondierenden Gelenkabschnitt zusammenwirkt und die gegenüberliegenden Gelenkseiten so ein schwenkbares Gelenk ausbilden.

Da die Gelenkimplantatkomponente nur zeitweilig, d. h. für die Dauer der Infektionsbehandlung, in das Knochengewebe eingesetzt ist, wird der Verbindungsabschnitt des Implantatschafts sowie der Verbindungsabschnitt des Gelenkabschnitts vorzugsweise als Gewindeverbindung ausgeführt. Diese ist insbesondere kostengünstig. Zudem lässt sich das Gelenkimplantat durch so einen Verbindungsabschnitt sowohl schnell und einfach montieren als auch schnell und einfach wieder zerlegen.

Alternativ oder ergänzend ist es auch möglich, den Verbindungsabschnitt als konische Verbindung vorzusehen. Eine derartige konische Verbindung kann dabei eine dichtende Funktion aufweisen.

Bei einer bevorzugten Ausführungsform definiert das mindestens eine Schaftrückspülloch einen Rückspülpfad und jedes Schaftspülloch einen Spülpfad, wobei der Rückspülpfad eine andere Ausrichtung aufweist als der Spülpfad und sich die Ausrichtungen zwischen jedem der Schaftspüllöchern und dem mindestens einen Schaftrückspülloch vorzugsweise um 45° bis 90° und noch bevorzugter um 60° bis 90° unterscheiden.

Der Spülpfad und der Rückspülpfad werden im Wesentlichen durch die Mittelachsen der Schaftspüllöcher bzw. die Mittelachse des mindestens einen Schaftrückspüllochs definiert.

Mit anderen Worten ist die Implantatkomponente bei dieser Ausführungsform so gestaltet, dass die Schaftspüllöcher das Arzneimittel in Richtungen abgeben, die sich von der Aufnahmerichtung des mindestens einen Schaftrückspüllochs für die Drainageflüssigkeit unterscheiden. Dies hat den Vorteil, dass bei einer Drainage ein zugeführtes Arzneimittel nicht direkt wieder aus dem Infektionsbereich abgeführt wird, sondern eine gewisse Verweildauer des Arzneimittels und damit dessen Wirkung gewährleistet werden kann. Die Verweildauer ist bei einer passiven Drainage am längsten und kann durch eine aktive Drainage verkürzt werden. Bei einer aktiven Drainage wird die Drainageflüssigkeit durch einen Unterdruck über die Rückspülöffnung wieder entfernt.

Das mindestens eine Schaftrückspülloch befindet in einem anderen Abschnitt des Implantatschafts als die Schaftspüllöcher. So kann sich zum Beispiel das mindestens eine Schaftrückspülloch in einem distalen Bereich, insbesondere bei dem distalen Ende des Implantatschafts befinden, während die Schaftspüllöcher am Umfang des Implantatschafts, insbesondere zumindest im mittleren und/oder proximalen Bereich des Implantatschafts angeordnet sind. Alternativ oder ergänzend ist es auch möglich, das mindestens eine Schaftrückspülloch an einer in Umfangsrichtung des Implantatschafts anderen Stelle vorzusehen als die Schaftspüllöcher.

Bei einer besonders bevorzugten Ausführungsform weist die Gelenkimplantatkomponente proximal zu den Schaftspüllöchern einen Verankerungsabschnitt mit einer Verankerungsfläche auf, über welche die Gelenkimplantatkomponente insbesondere mittels Knochenzement verankerbar ist. Die Verankerungsfläche der Gelenkimplantatkomponente erstreckt sich dabei bevorzugt im Wesentlichen in einer Richtung quer zu der Längsachse des Implantatschafts, d. g. quer zu der Implantationsrichtung der Gelenkimplantatkomponente.

Diese Ausführungsform ermöglicht eine ausreichend feste Verankerung der Gelenkimplantatkomponente, um zumindest ein eingeschränktes Ausmaß an Mobilität zu gewährleisten, und definiert zudem den Behandlungsraum für das Arzneimittel im Bereich des Implantatschafts. Insbesondere wird hierdurch zumindest ein Teil des Implantatschafts im implantierten Zustand, der die Schaftspüllöcher und das mindestens eine Schaftrückspüllochaufweist, in der Aussparung des Knochengewebes angeordnet und auf der Höhe des Verankerungsabschnitts abgedichtet. Folglich ergibt sich bei dieser Ausführungsform im Bereich des Implantatschafts ein Behandlungsraum, in dem die Infektion räumlich gezielter und effizienter bekämpft werden kann.

Außerdem wird bei einer Querausrichtung der Verankerungsfläche die Entfernung der Implantatkomponente vereinfacht, da die Verbindung zwischen Knochengewebe und Knochenzement einfach zugänglich und damit einfach trennbar ist.

Weiterhin ist der Verankerungsabschnitt vorzugsweise an einer Fläche des Gelenkabschnitts vorgesehen, die noch bevorzugter wie oben beschrieben sich in einer Richtung quer zur Längsachse des Implantatschafts erstreckt. Die Verankerungsfläche ist dabei der distalen Seite der Gelenkimplantatkomponente zugewandt.

Durch diese Anordnung des Verankerungsabschnitts wird dagegen vorgebeugt, dass bei Verwendung von Knochenzement dieser die Schaftabgabelöcher oder das mindestens eine Schaftrückspülloch blockiert.

Bei einer Ausführungsform weist die Oberfläche des Verankerungsabschnitts zur Unterstützung der Verankerung des Implantats eine Oberflächenstruktur, insbesondere Rippen und/oder Nuten, auf.

Eine derartige Oberflächenstruktur vergrößert die Kontaktfläche zum Knochenzement. Sie kann ausgeführt sein, um durch einen Formschluss zumindest gegen eine Relativbewegung entlang der Verankerungsfläche des Verankerungsabschnitts vorzubeugen. Es ist anzumerken, dass die Verwendung von Knochenzement zur Verankerung der Implantatkomponente bevorzugt wird, da eine solche Verankerung bei der vorliegenden Gelenkimplantatkomponente den Patienten zuverlässig und schnell mobilisieren kann. Diese Mobilisation bedeutet dabei sowohl einen Zugewinn an Lebensqualität als auch verbesserte Therapiemöglichkeiten in Bezug auf den Bewegungsapparat.

Bei einer bevorzugten Ausführungsform ist die Rückspülöffnung in dem Gelenkabschnitt vorgesehen und der Gelenkabschnitt weist einen Gelenkabschnittrückspüldurchgang auf, der die Rückspülöffnung mit dem Schaftrückspülkanal verbindet.

Auf diese Weise wird die Drainageflüssigkeit aus dem Infektionsbereich über das mindestens eine Schaftrückspülloch, den Schaftrückspülkanal, den Gelenkabschnittrückspüldurchgang und die Rückspülöffnung über eine aktive oder passive Drainage entfernt. Bei einer aktiven Drainage wird an der Rückspülöffnung ein Unterdruck angelegt, der die Drainageflüssigkeit ansaugt, während bei einer passiven Drainage Flüssigkeit durch Überdruck, Schwerkraft, das Prinzip der kommunizierenden Röhre oder Kapillarwirkung zu der Rückspülöffnung transportiert wird, um die Drainageflüssigkeit aus dem Patientenkörper zu entfernen. In beiden Fällen hat diese Anordnung einer Rückspülöffnung den Vorteil, dass die entnommene Flüssigkeit im Bereich des Implantationszugangs für die Gelenkimplantatkomponente entfernt wird und folglich kein weiterer Zugang erzeugt werden muss.

Bei einer weiteren bevorzugten Ausführungsform ist die Spülzuführöffnung in dem Gelenkabschnitt vorgesehen und der Gelenkabschnitt weist einen Gelenkabschnittspüldurchgang auf, der die Spülzuführöffnung mit dem Schaftspülkanal verbindet.

Bei dieser Ausführungsform wird das Arzneimittel über die Zufuhröffnung, den Gelenkabschnittspüldurchgang, den Schaftspülkanal und die Schaftspüllöcher dem entzündeten Bereich auf der Höhe des Implantatschafts zugeführt. Wie bei der Rückspülöffnung hat auch diese Anordnung den Vorteil des an dieser Stelle bereits vorhandenen Implantationszugangs.

Bei einer bevorzugten Ausführungsform weist der Gelenkabschnitt Gelenkabschnittspüllöcher auf, die mit der Spülzuführöffnung der Gelenkimplantatkomponente verbunden sind.

Durch diese Ausführungsform wird ermöglicht, auch einer Infektion im Gelenkbereich zu begegnen oder einer Ausbreitung einer Infektion in diesen Bereich vorzubeugen. Vorzugsweise sind diese Gelenkabschnittspüllöcher so angeordnet, dass sie durch die Artikulation des Gelenks nicht blockiert werden bzw. zu der Umgebung des Implantats geöffnet bleiben. Dementsprechend sind die Gelenkabschnittspüllöcher vorzugsweise so angeordnet, dass sie einen Spülpfad definieren, der im Wesentlichen senkrecht zu der Längsachse des Implantatschafts ausgerichtet ist.

Bei einer Ausführungsform weist der Gelenkabschnittspüldurchgang mindestens eine Verzweigung auf, welche die Spülzuführöffnung mit dem Schaftspülkanal und/oder den Gelenkabschnittspüllöchern verbindet.

Die mindestens eine Verzweigung dient dazu, sowohl dem Schaftspülkanal als auch den Gelenkabschnittspüllöchern Arzneimittel zuzuführen. So kann zum Beispiel eine Verzweigung den Arzneimittelstrom auf den Schaftspülkanal und mindestens ein Gelenkabschnittspülloch aufteilen. Zudem kann mindestens eine weitere Verzweigung vorgesehen sein, die der vorhergehenden Verzweigung nachgelagert ist und den Arzneimittelstrom zu mehreren Gelenkabschnittspüllöchern führt. Über die Anzahl der abgehenden Äste einer Verzweigung als auch die Anordnung der mindestens einen Verzweigung kann zudem die Aufteilung des Arzneimittelstroms und damit die Verabreichung des Arzneimittels gesteuert werden.

Bei einer besonders bevorzugten Ausführungsform weist der Gelenkabschnittspüldurchgang unterschiedliche Querschnittsflächen für eine Steuerung der Aufteilung des Arzneimittels zwischen dem Schaftspülkanal und den Gelenkabschnittspüllöchern auf.

Bei dieser Ausführungsform wird über eine gezielte Wahl der Querschnittsflächen der von der mindestens einen Verzweigung abgehenden Äste die Aufteilung des Volumenstroms, der das Arzneimittel enthält, definiert. So wird für den Ast, der das Arzneimittel in den Schaftspülkanal führt, vorzugsweise eine größere Querschnittsfläche gewählt als die Querschnittsfläche, die zu den Gelenkabschnittspüllöchern führt. Folglich wird bevorzugt, die Querschnittsflächen so auszulegen, dass ein größerer Anteil des Arzneimittelstroms dem Schaftspülkanal zugeführt wird.

Bei einer weiteren bevorzugten Ausführungsform weisen die Schaftspüllöcher untereinander unterschiedliche Querschnittsflächen für eine Steuerung der Abgabe des Arzneimittels auf.

Folglich können unterschiedliche Querschnittsflächen zwischen den Schaftspüllöchern vorgesehen sein. Beispielsweise ist es möglich, die Schaftspüllöcher, die weiter von der Spülzuführöffnung entfernt sind, mit einer größeren Querschnittsfläche zu versehen als die Schaftspüllöcher, die näher an der Spülzuführöffnung liegen. Damit kann eine gleichmäßigere Abgabe des Arzneimittels über die derart ausgebildeten Schaftspüllöcher erreicht werden. In diesem Zusammenhang beziehen sich die Begriffe "weiter entfernt" und "näher" auf die Entfernung innerhalb der Gelenkimplantatkomponente von der Spülzuführöffnung aus.

Eine Derartige Auslegung von Querschnittsflächen und/oder Verzweigungen ist für jegliche Aufnahmelöcher und/oder Abgabelöcher möglich. Beispielsweise kann Ähnliches für den Querschnitt des Strömungspfads gelten, über den durch das mindestens eine Schaftrückspülloch aufgenommene Drainageflüssigkeit aus der Implantatkomponente abgeführt wird.

Zudem ist es besonders bevorzugt, dass das mindestens eine Schaftrückspülloch einen größeren Querschnitt aufweist als jedes der Schaftspüllöcher. Auf diese Weise wird einem Verstopfen des Rückführströmungspfads durch Rückstände aus dem Infektionsbereich vorgebeugt. Insbesondere ist der minimale Querschnitt des Rückführpfads 2, 3 oder 5 Mal bis zu 10 oder 15 Mal so groß wie der minimale Querschnitt des Spülzuführpfads.

Bei einer besonders bevorzugten Ausführungsform weist der Gelenkabschnitt ein anbringbares Gelenkelement für eine Artikulation mit einer weiteren Gelenkimplantatkomponente auf.

Bei dieser Ausführungsform weist der Gelenkabschnitt anstelle einer integral ausgeführten Gelenkfläche ein anbringbares Gelenkelement auf, das wiederum eine Gelenkfläche für eine Artikulation mit einer weiteren Gelenkimplantatkomponente bereitstellt. Diese Ausführungsform ermöglicht eine noch bessere Anpassung des Gelenksabschnitts und zudem auch eine Implantation, bei welcher die Gelenkfläche der Gelenkimplantatkomponente erst montiert wird, nachdem der Implantatschaft in die Aussparung im Knochengewebe eingebracht worden ist.

Des Weiteren wird ein Gelenkimplantat nach Anspruch 12 bereitgestellt, das zwei Gelenkimplantatkomponenten aufweist, wobei die erste Gelenkimplantatkomponente eine erste Gelenkfläche und die zweite Gelenkimplantatkomponente eine zweite Gelenkfläche aufweist und die Gelenkflächen der ersten und zweiten Gelenkimplantatkomponenten komplementär zueinander ausgebildet sind und in Kontakt miteinander relativ zueinander verschwenkbar sind.

Bei einem derartigen Gelenkimplantat werden beide Gelenkseiten durch Gelenkimplantatkomponenten ersetzt, die eine Arzneimittelzufuhr ermöglichen. Wie bei einer einzelnen Gelenkimplantatkomponente wird hier, wenn auch beidseitig, eine vorhandene Infektion bzw. Entzündung umliegenden Gewebes gezielt und effizient behandelt. Gleichzeitig wird eine erhöhte Mobilität des Patienten erreicht, in dem die erste und zweite Gelenkimplantatkomponente zueinander verschwenkbar sind.

Bei einer Ausführungsform ist die erste Gelenkfläche und/oder die zweite Gelenkfläche als Teil eines ersten beziehungsweise zweiten anbringbaren Gelenkelements ausgeführt, wobei vorzugsweise eine Gelenkbolzen die erste Gelenkfläche und ein Durchgangsloch die zweite Gelenkfläche ausbildet.

Wie oben beschrieben, ermöglicht eine solche Ausführungsform eine erhöhte Anpassungsfähigkeit an die Situation eines Patienten sowie eine leichtere Implantation der Gelenkimplantatkomponenten.

Vorzugsweise ist das Gelenkimplantat dieser Ausführungsform mit einem Gelenkbolzen und einer Aufnahme für den Gelenkbolzen als Scharniergelenk ausgeführt. Ein solches Gelenk weist einen einfachen Aufbau auf und ist ausreichend belastbar, um dem Patienten eine bessere Beweglichkeit zu ermöglichen.

Weiterhin kann eine der Gelenkimplantatkomponenten einen Arthrodesebolzen und die andere der Gelenkimplantatkomponenten eine Arthrodesebolzenaufnahme aufweisen, wobei der Arthrodesebolzen in die Arthrodesebolzenaufnahme einführbar ist, um die Gelenkimplantatkomponenten vorzugsweise in einer gestreckten Stellung zu verriegeln.

Eine Verriegelung des Gelenkimplantats führt zu einer Arthrodese und damit einer Ruhigstellung des Gelenks, womit das umliegende Gewebe geschont werden kann. Der Arthrodesebolzen ist vorzugsweise quer, insbesondere im Wesentlichen senkrecht, zur Längsachse des Implantatschafts in den Gelenkabschnitt einführbar. Dadurch ist es möglich, das Gelenk bedarfsweise über eine Arthrodese ruhig zu stellen oder auch wieder zu lösen.

Bei einer besonders bevorzugten Ausführungsform des Gelenkimplantats weist das zweite Gelenkelement einen Einsetzabschnitt auf, der in eine Einführöffnung und einen Führungsabschnitt mit einem Hinterschnitt einsetzbar ist, wobei die Einführöffnung und der Führungsabschnitt in der zweiten Gelenkimplantatkomponente auf einer Seite ausgebildet sind, die der ersten Gelenkimplantatkomponente zugewandt ist, und die zweite Gelenkimplantatkomponente vorzugsweise eine Arretierungseinrichtung aufweist, welche den in den Führungsabschnitt eingesetzten Einsetzabschnitt arretiert.

Dadurch, dass das Gelenkelement mittels eines Einsetzabschnitts in eine Aussparung einführbar ist, die durch eine Einführöffnung und einen Führungsabschnitt ausgebildet wird. Der Führungsabschnitt weist dabei einen Hinterschnitt auf, über den eine einfache Kopplung des Gelenkelements mit dem entsprechenden Gelenkabschnitt möglich ist. Nach dem Einsetzen des Einsetzabschnitts in die Einsetzöffnung, kann das Gelenkelement unter den Hinterschnitt geführt werden. Wenn der Einsetzabschnitt zumindest abschnittsweise im Hinterschnitt angeordnet ist, ist das Gelenkelement in der zu der Einsetzrichtung entgegengesetzten Richtung verriegelt. Mittels der Arretierungsvorrichtung ist es dabei möglich, einer unbeabsichtigten Entriegelung des zweiten Gelenkelements vorbeugen.

### KURZE BESCHREIBUNG DER FIGUREN

Mit Hilfe der folgenden Figuren und Beschreibung werden Ausführungsbeispiele für ein besseres Verständnis der vorliegenden Erfindung genauer beschrieben. Hierfür werden die in den Figuren ersichtlichen Merkmale mit Bezugszeichen gekennzeichnet. Dabei werden bei unterschiedlichen Ausführungsbeispielen im Wesentlichen gleiche Bezugszeichen verwendet, sofern sich die Merkmale dieser Ausführungsbeispiele gleichen oder eine gleiche Wirkung erzielen.
Figur 1 veranschaulicht eine erste Ausführungsform eines Gelenkimplantats im gestreckten Zustand, das für eine Verabreichung eines Arzneimittels eingerichtet ist;
Figur 2 zeigt eine zweite Ausführungsform eines Gelenkimplantats im gebeugten Zustand für eine Verabreichung eines Arzneimittels;
Figur 3a ist eine Darstellung einer Ausführungsform eines Gelenkabschnitts einer Gelenkimplantatkomponente mit einem montierten Gelenkelement;
Figur 3b veranschaulicht eine Ausführungsform eines Gelenkabschnitts einer weiteren Gelenkimplantatkomponente, die mit der Gelenkimplantatkomponente aus Figur 3a kombinierbar ist;
Figur 3c ist eine dreidimensionale Ansicht, welche die Montage des Gelenkabschnitts der Figur 3a einer ersten Gelenkimplantatkomponente mit dem Gelenkabschnitt der Figur 3b einer zweiten Gelenkimplantatkomponente veranschaulicht;
Figur 4a ist eine dreidimensionale Ansicht von einer der in Figur 3c dargestellten Gelenkimplantatkomponenten;
Figur 4b ist eine Schnittansicht der Gelenkimplantatkomponente aus Figur 4a zur Verdeutlichung der Arzneimittelabgabe und Arzneimittelrückführung;
Figur 4c ist eine Schnittansicht des Gelenkabschnitts der Gelenkimplantatkomponente aus Figur 4b auf der Höhe des Kanalsystems für eine Arzneimittelabgabe und -rückführung;
Figur 5a ist eine dreidimensionale Darstellung der anderen in Figur 3c dargestellten Gelenkimplantatkomponenten;
Figur 5b ist eine Schnittansicht der Gelenkimplantatkomponente aus Figur 5a, die ein Teil des Kanalsystems zur Arzneimittelabgabe und Arzneimittelrückführung darstellt;
Figur 5c ist eine Schnittansicht des Gelenkabschnitts der Gelenkimplantatkomponente aus den Figuren 5a und 5b, der das Kanalsystem für die Arzneimittelabgabe und -rückführung sowie einen Teil einer Arthrodeseeinrichtung veranschaulicht;
Figur 6a und Figur 6b veranschaulichen die strukturellen Merkmale zur Verankerung des ersten und zweiten Gelenkabschnitts des in den vorangegangenen Figuren dargestellten Gelenkimplantats;
Figur 7 ist eine Schnittansicht einer noch weiteren Ausführungsform einer Gelenkimplantatkomponente für die Abgabe und Rückführung eines Arzneimittels;
Figur 8a ist eine dreidimensionale Ansicht einer weiteren Ausführungsform einer Gelenkimplantatkomponente für die Abgabe und Rückführung eines Arzneimittels; und
Figur 8b ist eine dreidimensionale Ansicht des inneren Kanalsystems zur Abgabe und Rückführung eines Arzneimittels im implantierten Zustand der Gelenkimplantatkomponente.

### AUSFÜHRLICHE BESCHREIBUNG DER AUSFÜHRUNGSFORMEN

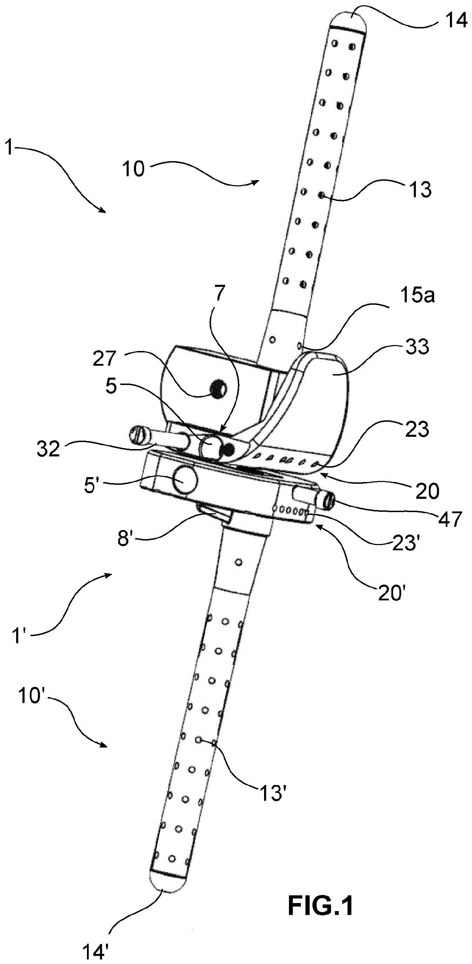

Figur 1 zeigt eine erste Ausführungsform eines Gelenkimplantats, das für eine Verabreichung eines Arzneimittels zu einem entzündeten Bereich sowie für eine Drainage des entzündeten Bereichs eingerichtet ist. Folglich kann mit einem derartigen Gelenkimplantat, das die in Figur dargestellten Gelenkimplantatkomponenten 1 und 1' aufweist, nach dessen Implantation entzündetes Gewebe in der Umgebung des Gelenkimplantats behandelt werden. Hierfür kann mit Hilfe eines Kanalsystems innerhalb der Gelenkimplantatkomponenten 1, 1' ein Arzneimittel zugeführt werden, das gegen die Entzündung und/oder deren Folgen wirkt. Beispiele solcher Arzneimittel sind Entzündungshemmer und Antibiotika.

Das Arzneimittel wird in gelöster Form oder als Suspension über das Kanalsystem des Gelenkimplantats zu dem entzündeten Bereich transportiert. Mittels einer Drainage kann zudem aus dem entzündeten Bereich Drainageflüssigkeit über ein weiteres Kanalsystem innerhalb der Gelenkimplantatkomponenten 1, 1' abgeführt werden. Die Drainageflüssigkeit enthält Rückstände aus dem entzündeten Bereich, sodass mit den Gelenkimplantatkomponenten 1, 1' auch gegen eine systemische Ausbreitung der Infektion vorgebeugt werden kann.

Die in Figur 1 dargestellte beispielhafte Ausführungsform eines Gelenkimplantats ist als Ersatz für ein vollwertiges Implantat vorgesehen, das aufgrund einer Infektion und zu dessen Behandlung vollständig explantiert wird.

Abweichend von einem vollständigen Ersatz ist es ebenso möglich, nur eine Seite eines vollwertigen Gelenkimplantats durch eine Gelenkimplantatkomponente zur Verabreichung eines Arzneimittels zu ersetzen. Dabei wird der Gelenkabschnitt an die nicht entnommene Implantatkomponente des vollwertigen Gelenkimplantats angepasst, sodass die Funktion des künstlichen Gelenks im Wesentlichen erhalten werden kann. Damit kann dem Patienten eine Mobilität ermöglicht werden, die zum einen die Therapie unterstützen kann und zum anderen dem Patienten ermöglicht, einfache Bewegungen auszuführen.

Das in Figur 1 dargestellte Beispiel eines Gelenkimplantats, das die Gelenkimplantatkomponenten 1, 1' aufweist, ist als Ersatz für ein vollwertiges Kniegelenkimplantat vorgesehen. Dabei ersetzt die Gelenkimplantatkomponente 1 die femorale Kniegelenkkomponente und die Gelenkimplantatkomponente 1' die tibiale Kniegelenkkomponente des vollwertigen Gelenkimplantats. Der strukturelle Aufbau und/oder die Funktion der beiden Gelenkimplantatkomponenten 1 und 1' ist dabei in Teilen im Wesentlichen gleich. Im Folgenden wird deswegen im Allgemeinen auf beide Gelenkkomponenten gleichzeitig Bezug genommen. Zudem sind nachfolgend Merkmale zusammengehöriger Gelenkimplantatkomponenten mit gleichem strukturellem Aufbau und/oder gleicher Funktion im Allgemeinen mit gleichem Bezugszeichen mit und ohne Apostroph gekennzeichnet.

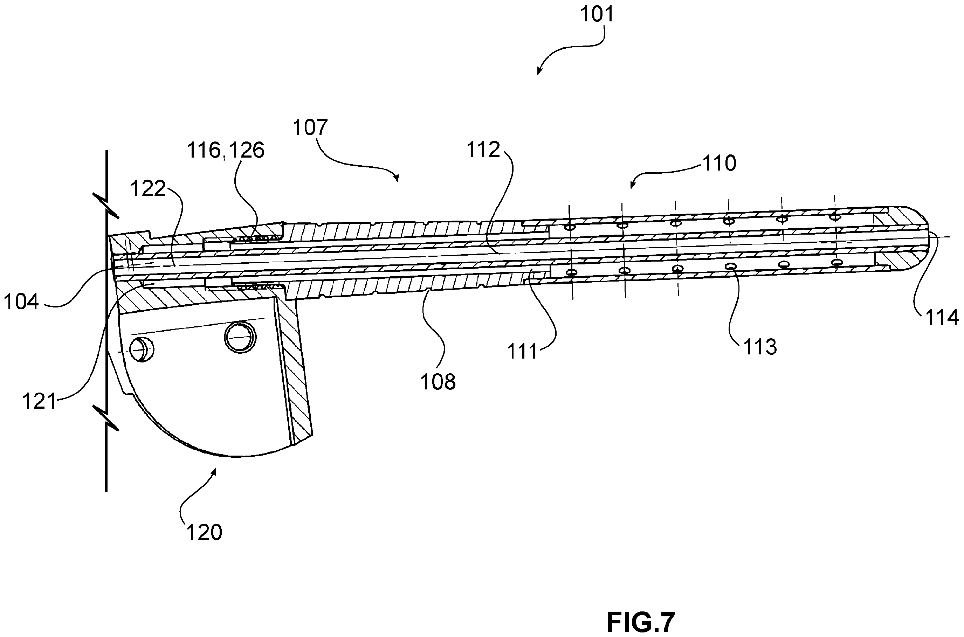

Als zeitweiliger Ersatz für ein vollwertiges Gelenkimplantat ausgebildete Gelenkimplantatkomponenten können selbstverständlich auch eingesetzt werden, um Implantatkomponenten vollwertiger Gelenkimplantate anderer Gelenke zu ersetzen. Beispielsweise wird weiter unten unter Bezugnahme auf die Figuren 7a und 7b eine Ausführungsform beschrieben, bei welcher eine Gelenkimplantatkomponente 101 für eine temporäre Behandlung der femoralen Implantatkomponente eines nicht dargestellten vollwertigen Hüftgelenkimplantats eingerichtet ist. Mit anderen Worten ist die vorliegende Offenbarung auf Gelenkimplantatkomponenten verschiedener Gelenke anwendbar, wie zum Beispiel auf Gelenkimplantatkomponenten von Kniegelenken, Hüftgelenken Schultergelenken, Ellenbogengelenken, Sprunggelenken, Handgelenken oder Wirbelsäulengelenken.

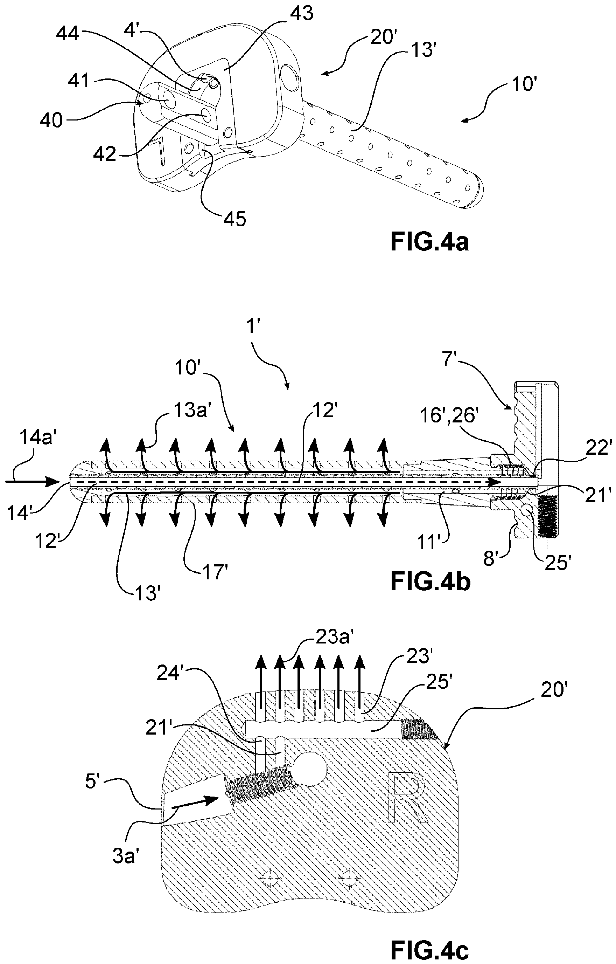

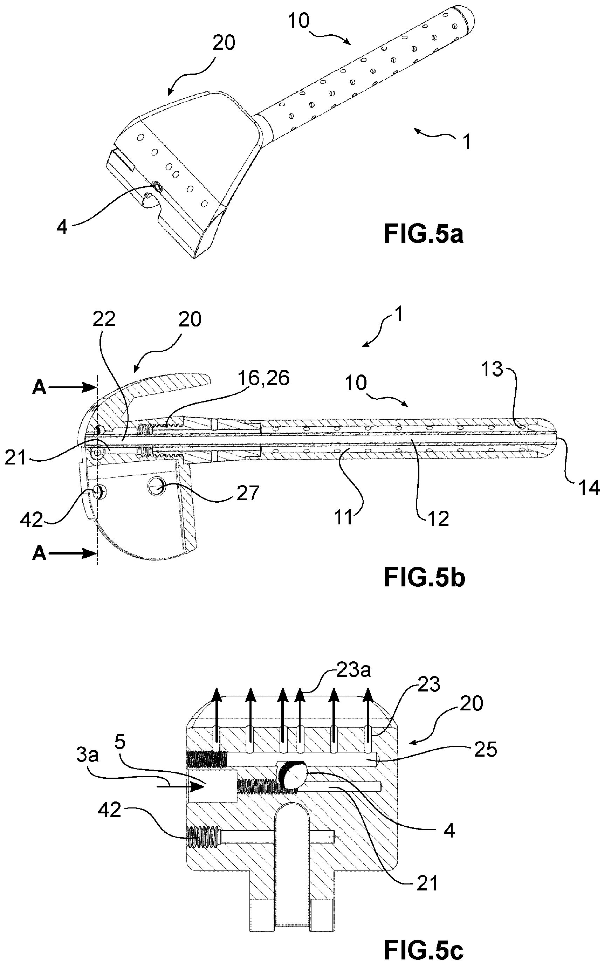

Die in Figur 1 dargestellten Gelenkimplantatkomponenten 1, 1' weisen jeweils einen Implantatschaft 10, 10' und einen Gelenkabschnitt 20, 20' auf. Der Implantatschaft 10, 10' und der Gelenkabschnitt 20, 20' können im Wesentlichen einteilig ausgeführt sein, sind aber vorzugsweise über einen Verbindungsabschnitt 16 lösbar miteinander verbunden (vgl. Figur 4). Wie oben beschrieben und in Figur 4 dargestellt, können der Verbindungsabschnitt 16 des Implantatschafts 10, 10' und der Verbindungsabschnitt 26 des Gelenkabschnitts 20. 20' mit einem Gewinde ausgebildet sein (Figuren 4b und 5b). Dementsprechend sind der Implantatschaft 10, 10` und der Gelenkabschnitt 20, 20' über eine Gewindeverbindung montierbar und zerlegbar. Wie oben bereits ausgeführt, kann alternativ oder ergänzend eine Konusverbindung eingesetzt werden, die zudem eine dichtende Funktion aufweisen kann.

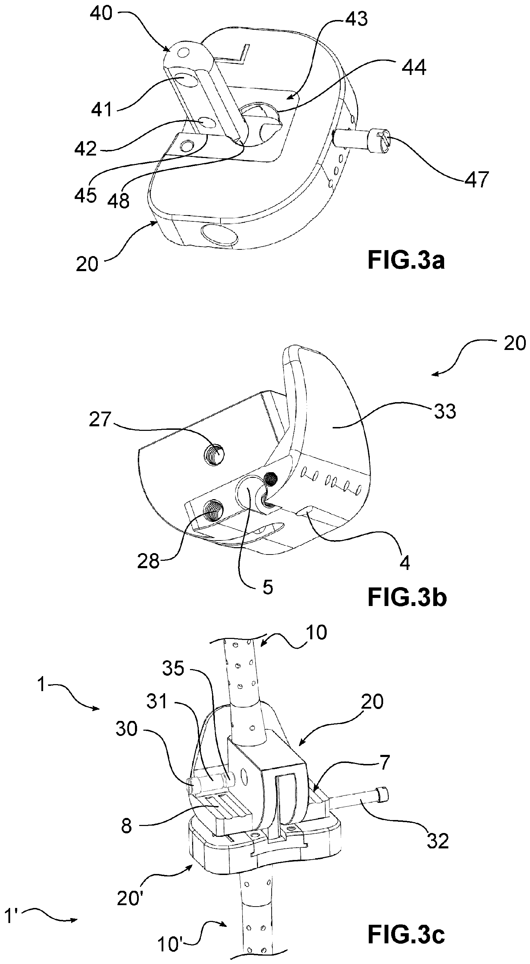

Die zwei Gelenkimplantatkomponenten 1 und 1' sind gelenkig miteinander gekoppelt. Dabei wirken die Gelenkabschnitte 20 und 20' so zusammen, dass sie relativ zueinander verschwenkbar sind. Bei dem in Figur 1 dargestellten Ausführungsbeispiel wird ein relatives Verschwenken der Gelenkimplantatkomponenten 1 und 1' über ein erstes Gelenkelement 30 und ein zweites Gelenkelement 40 ermöglicht (vgl. Figur 3). Dabei wird vorzugsweise eine Verschwenkbarkeit in einem Bereich von 0° bis 100° von der gestreckten Haltung aus vorgesehen.

Das erste Gelenkelement 30 ist bei dem in Figur 1 gezeigten Ausführungsbeispiel als stabförmiger Gelenkbolzen ausgeführt, mittels dem die Gelenkimplantatkomponenten 1 und 1' ein Scharniergelenk ausbilden. Wie in Figur 3b veranschaulicht, kann das erste Gelenkelement 30 einen Gewindeabschnitt 35 aufweisen. Über diesen kann das erste Gelenkelement 30 an dem Gelenkabschnitt 20 angebracht werden, der hierfür eine Gelenkelementaufnahme 27 mit einem Gewinde aufweist.

Die Kinematik des Gelenkimplantats kann bzw. die Gelenkimplantatkomponenten 1 und 1` können jedoch auch an andere Gelenke angepasst werden, wie zum Beispiel an das Kugelgelenk eines Schultergelenkimplantats oder an das Kugel- oder Nussgelenk eines Hüftgelenkimplantats.

Das in den Figuren veranschaulichte Kniegelenkimplantat kann wahlweise durch die Verwendung eines Arthrodesebolzens 32 auch als Arthrodeseimplantat eingesetzt werden (siehe Figur 3c). Mit anderen Worten sind die Gelenkimplantatkomponenten 1 und 1` mittels eines solchen Arthrodesebolzens als Arthrodeseimplantat fixierbar. Durch die hierdurch hervorgerufene Versteifung wird das Bein ruhiggestellt und dadurch geschont.

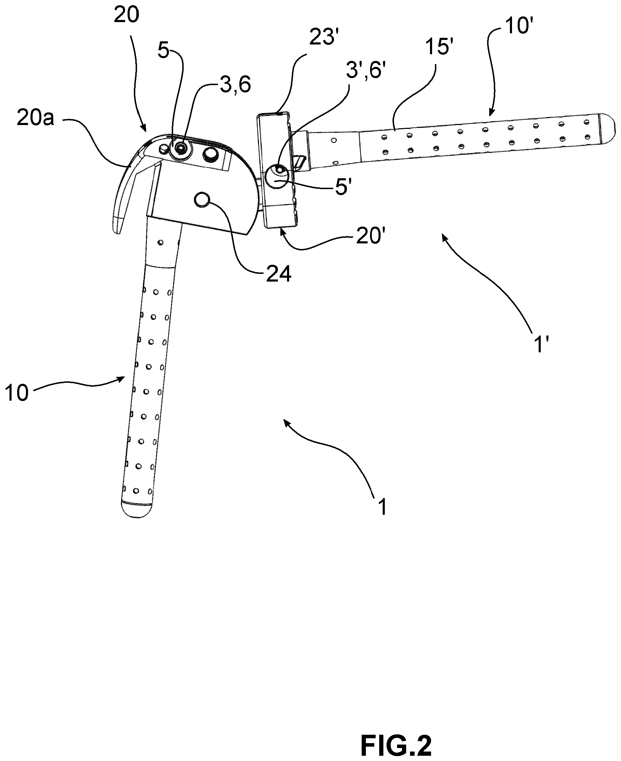

Weiterhin ist bei der Gelenkimplantatkomponente 1, 1' mindestens eine Spülzuführöffnung 3, 3' vorgesehen, über die eine ein Arzneimittel enthaltene Flüssigkeit in die Gelenkimplantatkomponente 1, 1' eingeführt werden kann (siehe Figur 2). Die Spülzuführöffnung 3, 3' ist bei der in Figur 1 dargestellten Ausführungsform als Kniegelenk vorzugsweise seitlich vorgesehen. Mit anderen Worten befindet sich die Spülzuführöffnung 3, 3' im implantierten Zustand auf der lateralen oder medialen Seite.

Zum Abführen von Drainageflüssigkeit ist bei der Gelenkimplantatkomponente 1, 1' eine Rückspülöffnung 4 vorgesehen (vgl. Figuren 4a und 5a).

Weiterhin kann der Gelenkabschnitt 20, 20' Gelenkabschnittspüllöcher 23, 23' aufweisen. Über diese Gelenkabschnittspüllöcher 23, 23' kann das über die Spülzuführöffnungen 3, 3' zugeführte Arzneimittel auf Gelenkhöhe in die Umgebung des Implantats abgegeben werden. Hierfür weist der Gelenkabschnitt 20, 20' ein Kanalsystem mit entsprechenden Verzweigungen 24, 24' auf, das weiter unten ausführlicher beschrieben wird (vgl. Figuren 4c und 5c).

Der sich an den Gelenkabschnitt 20, 20' anschließende Implantatschaft 10, 10' weist eine längliche Form auf. Im distalen Bereich des Schafts 10, 10' ist eine Mehrzahl von Schaftspüllöchern 13, 13' am Umfang des Schafts 10, 10' angeordnet. Die Schaftspüllöcher 13, 13' stehen mit der Spülzuführöffnung 3, 3' in Fluidverbindung, sodass ein durch die Spülzuführöffnung 3, 3' zugeführtes Arzneimittel über die Schaftspüllöcher 13, 13' an die Umgebung des Implantatschafts 10, 10' abgegeben werden kann. Insbesondere ist hierfür in dem Schaft 10, 10' ein Schaftspülkanal 11 ausgebildet, der auf seiner proximalen Seite mit der Spülzuführöffnung 3, 3' über einen Gelenkabschnittspüldurchgang 21, 21' in Fluidverbindung steht (siehe Figuren 4b und 5b). Die Schaftspüllöcher 13, 13' sind vorzugsweise am Umfang des Implantschafts 10, 10` vorgesehen, sodass sie über im Wesentlichen radiale Strömungspfade Flüssigkeit zur Außenseite des Implantatschafts 10, 10' abgeben. Auf diese Weise kann entzündetes Gewebe, insbesondere Knochengewebe, in der Umgebung des Schafts 10, 10' durch Verabreichung eines Arzneimittels auf effektive Weise behandelt werden.

Weiterhin befindet sich am distalen Ende des Schafts 10, 10' der in Figur 1 veranschaulichten beispielhaften Ausführungsform ein Schaftrückspülloch 14, 14'. Alternativ können auch mehrere Schaftrückspüllöcher 14, 14' am Schaft 10, 10' vorgesehen sein, um Drainageflüssigkeit aufzunehmen. Jedoch wird eine geringe Anzahl an Schaftrückspüllöchern 14, 14` und insbesondere nur ein Schaftrückspülloch 14, 14` bevorzugt, um die Drainage möglichst gezielt durchführen zu können.

Dadurch, dass das Schaftrückspülloch 14, 14' am distalen Ende des Schafts 10, 10' angeordnet ist und somit keinem Knochengewebe gegenüberliegt, kann die Drainageflüssigkeit gezielt und auf einfache Weise in den Schaftrückspülkanal 12, 12` (siehe Figuren 4b und 5b) gelangen. Da der Schaft 10, 10' dafür vorgesehen ist, in einem Röhrenknochen implantiert zu sein, sammeln sich Körperflüssigkeit und Rückstände des zugeführten Arzneimittels insbesondere im Markraum des Röhrenknochens. Folglich ist es vorteilhaft, das Schaftrückspülloch 14, 14' so vorzusehen, dass sein Rückspülpfad 14a, 14a` durch den Markraum des Röhrenknochens verläuft.

Nachdem über das Schaftrückspülloch 14, 14' Drainageflüssigkeit in den Schaft 10, 10' gelangt ist, wird sie dort in dem Schaftrückspülkanal 12, 12` innerhalb des Schafts 10, 10' in Richtung der Rückspülöffnung 4, 4' auf der Höhe des Gelenkabschnitts 20, 20' transportiert und kann dort nach au-ßen hin abgeführt werden.

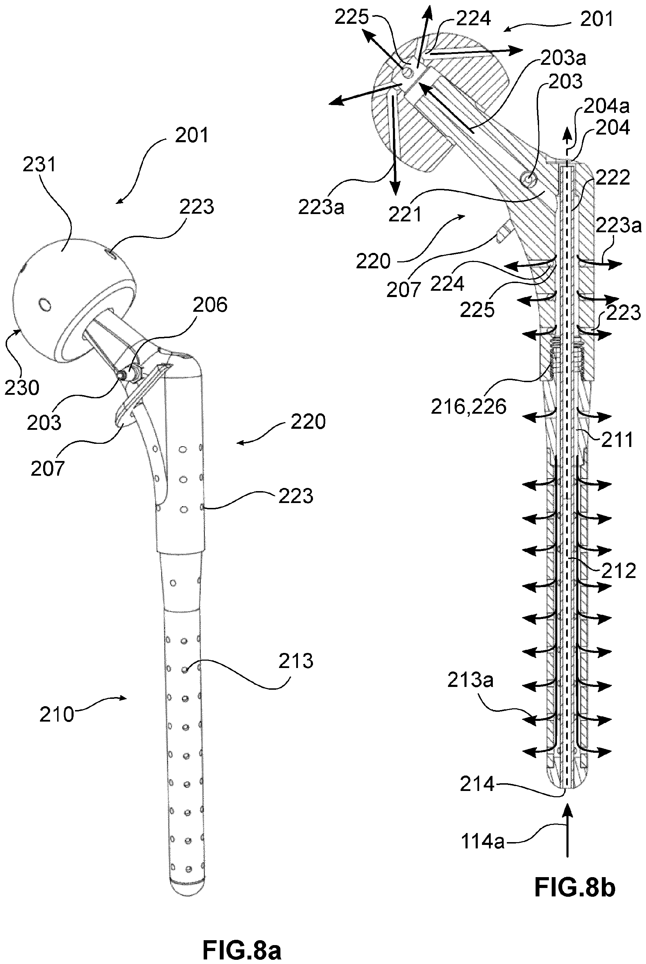

Wie in den Figuren 4b und 5b dargestellt und oben erwähnt, ist das Schaftrückspülloch 14, 14' vorzugsweise so ausgebildet, dass der Rückspülpfad 14a, 14a` eine andere Ausrichtung aufweist als jeder der Spülpfade 13a` der Schaftspüllöcher 13, 13' (vgl. auch Figur 8b). Der Rückspülpfad 14a und die Spülpfade 13a werden durch die Längsachse des Schaftrückspüllochs 14 bzw. die Längsachsen der Schaftspüllöcher 13, 13' definiert.

Wie der Figur 1 weiterhin zu entnehmen ist, weist die Implantatkomponente 1, 1' einen Verankerungsabschnitt 7, 7' auf, der dazu dient, die Gelenkimplantatkomponente 1, 1' im Knochengewebe zu verankern. Der Verankerungsabschnitt 7, 7' kann an dem Gelenkabschnitt 20, 20' (siehe Figuren 1 bis 6 und 8) und/oder dem Implantatschaft 10, 10` (siehe Figur 7) vorgesehen sein.

Für eine verbesserte Verankerung der Implantatkomponente 1, 1' kann der Verankerungsabschnitt 7, 7' eine Verankerungsstruktur 8, 8` aufweisen. Diese Verankerungsstruktur 8, 8' kann so ausgeführt sein, dass sie eine Verankerung der Gelenkimplantatkomponente 1, 1' mittels eines Formschlusses unterstützt.

Vorzugsweise wird die Gelenkimplantatkomponente 1, 1' durch Knochenzement im Knochengewebe eines Patienten verankert. Dies hat die oben beschriebenen Vorteile einer Abdichtung des zu behandelnden Gewebebereichs in Richtung des Gelenkabschnitts 20, 20' und einer schnellen und zuverlässigen Fixierung der Gelenkimplantatkomponente 1, 1' über den Implantatschaft 10, 10'.

Zum Beispiel weist die in den Figur 1 dargestellte Ausführungsform eines Gelenkimplantats einen Verankerungsabschnitt 7, 7' mit einer Verankerungsstruktur 8, 8' auf, welche einen Formschluss zwischen dem Verankerungsabschnitt 7, 7' und umliegendem Knochengewebe unterstützt. Dies dient dazu, gegen eine unbeabsichtigte Lockerung der Implantatkomponente 1, 1' vorzubeugen.

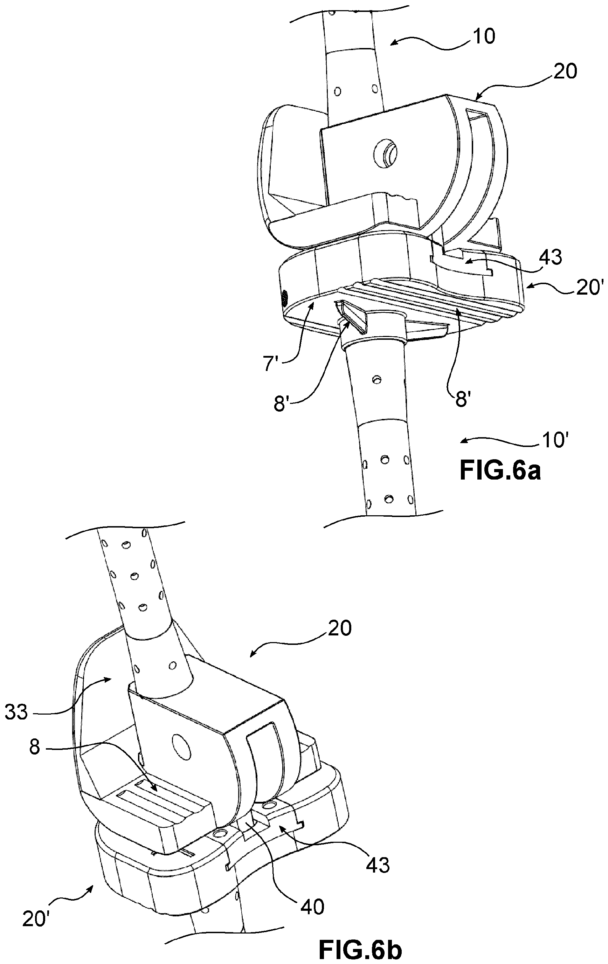

Wie in Figur 6a veranschaulicht, kann der Verankerungsabschnitt 7' des Gelenkabschnitts 20' hierfür eine Verankerungsstruktur 8' mit Rippen und Nuten aufweisen.

Bei der gezeigten beispielhaften Ausführungsform in Figur 6b weist der Gelenkabschnitt 20 ebenfalls einen Verankerungsabschnitt 7 auf. Dessen Verankerungsstruktur 8 wird durch Nuten unterstützt. Die Verankerungsstruktur 8 vergrößert zum einen die Kontaktfläche für eine Verankerung und ermöglicht zum anderen den zuvor bereits erwähnten Formschluss.

Es hat sich gezeigt, dass eine Verankerung auf diese Weise für die Behandlung mit den offenbarten Gelenkimplantaten vollständig ausreicht. Da hierbei im Wesentlichen keine Verankerung mit Knochenzement auf der Höhe des Schafts stattfindet, ist das Gelenkimplantat relativ einfach entnehmbar und das Knochengewebe im Schaftbereich wird geschont.

Figur 7 zeigt diesbezüglich eine weitere Ausführungsform einer Gelenkimplantatkomponente 101. Bei dieser Gelenkimplantatkomponente 1 ist ein Verankerungsabschnitt 107 an dem Implantatschaft 110 vorgesehen, der eine Verankerungsstruktur 108 aufweisen kann, die vorzugsweise als mindestens eine quer zur Implantationsrichtung verlaufende Aussparung ausgebildet ist. Bei der in Figur 7 veranschaulichten Ausführungsform wird die Verankerungsstruktur 108 insbesondere durch umlaufende Nuten ausgebildet.

Wiederum bezugnehmend auf Figur 1, wird der Außendurchmesser des Implantatschafts 10, 10' vorzugsweise kleiner gewählt als der Innendurchmesser des Röhrenknochens, um so eine Blockade der Schaftspüllöcher 13, 13' zu vermeiden und um die Abgabe des Arzneimittels über die Schaftspüllöcher 13, 13' zu erleichtern. Der Implantatschaft 10, 10` dient folglich im Wesentlichen zum Führen der Gelenkimplantatkomponente 1, 1` in einem Röhrenknochen und nicht zum Erzeugen eines Presssitzes zur Verankerung.

Bei einer Verankerung eines Implantatschafts 110 auf dessen proximalen Seite über einen wie in Figur 7 gezeigten Verankerungsabschnitt 107 ist es allerdings von Vorteil, den dortigen Durchmesser des Implantatschafts 110 unter Berücksichtigung eines Spalts für den Knochenzement an den Innendurchmesser des Röhrenknochens anzupassen. Aus diesem Grund ist der Durchmesser des auf der proximalen Seite des Implantatschafts 110 angeordneten Verankerungsabschnitts 107 vorzugsweise größer als der distale Abschnitt des Implantatschafts 110, wo sich die Schaftspüllöcher 113 und das mindestens eine Schaftrückspülloch 114 befinden. Wie in Figur 8 dargestellt, verjüngt sich der Implantatschaft 10, 10' in der Richtung von proximal nach distal vorzugsweise konisch.

In Figur 2 wird die Ausführungsform des Gelenkimplantats statt im gestreckten Zustand in einem gebeugten Zustand dargestellt. Die Gelenkimplantatkomponenten 1, 1` des Gelenkimplantats weisen vorzugsweise einen Bewegungsumfang (ROM - Range of Motion) in einem Bereich von 0° bis 100° auf.

Wie oben beschrieben, kann bei dem Gelenkimplantat eine Verriegelung mittels eines Arthrodesebolzens 32 vorgesehen sein. Diese Verriegelung wird bevorzugt in einer im Wesentlichen gestreckten Stellung der Gelenkimplantatkomponenten 1, 1` vorgenommen, d. h. in einer Stellung in einem Bereich von 0° bis 10°, 0° bis 5° und insbesondere 0°. Hierfür wird der Arthrodesebolzen 32 in ein Verriegelungsloch 28 des Gelenkabschnitts 20 und eine Arthrodesebolzenaufnahme 42 des anderen Gelenkabschnitts 20` eingeführt (Figuren 1, 3a und 3c). Das Verriegelungsloch 28 und der Arthrodesebolzen sind vorzugsweise mit einem Gewinde versehen, um den Arthrodesebolzen 32 im Verriegelungszustand zu sichern.

Weiterhin weist der Gelenkabschnitt 20 der in Figur 2 dargestellten Ausführungsform einen Patellaabschnitt 33 auf, an dem eine möglicherweise vorhandene Patella entlang gleiten kann. Der Patellaabschnitt kann auch bei der Fixierung des Gelenkabschnitts 20 unterstützen sowie das Gelenkimplantat schützen.

Wie zum Beispiel in Figur 2 dargestellt, sind die Spülzuführöffnungen 3, 3' der Gelenkimplantatkomponenten 1, 1` vorzugsweise zurückgesetzt, indem der Anschluss 6 mit der Spülzuführöffnung 3, 3' in einer Anschlussaussparung 5, 5' aufgenommen ist. Als Ergebnis steht die Spülzuführöffnung an der Außenkontur der Gelenkabschnitte 20, 20` im Wesentlichen nicht hervor. Hierdurch kann einer Verletzung umliegenden Gewebes durch die Struktur der Spülzuführöffnungen 3, 3 vorgebeugt werden.

Die Figuren 3a, 3b und 3c veranschaulichen insbesondere die gelenkige Verbindung zwischen den Gelenkabschnitten 20 und 20' der Gelenkimplantatkomponenten 1 und 1'. Bei der in diesen Figuren veranschaulichten Ausführungsform bildet das zweite Gelenkelement 40 des Gelenkabschnitts 20' zusammen mit dem ersten Gelenkelement 30 des Gelenkabschnitts 20, wie oben bereits erwähnt, ein Scharniergelenk aus (vgl. Figuren 1 und 2) .

Hierfür ist in dem zweiten Gelenkelement 40 ein mediallaterales Durchgangsloch vorgesehen, das die Gelenkfläche 42 des zweiten Gelenkelements 40 ausbildet (siehe Figur 3a). Im zusammengebauten Zustand des Gelenkimplantats wirkt die zweite Gelenkfläche 42 des zweiten Gelenkelements 40 mit einer ersten Gelenkfläche 31 des ersten Gelenkelements 30 zusammen.

Bei der in den Figuren 3a, 3b und 3c veranschaulichten Ausführung eines Kniegelenkimplantats ist das erste Gelenkelement 30 des Gelenkabschnitts 20 als Gelenkbolzen ausgeführt. Dieser ist über das die zweite Gelenkfläche 41 ausbildende Durchgangsloch durch das zweite Gelenkelement 40 durchführbar.

Für eine Montage der Gelenkabschnitte 20 und 20' werden zunächst das Durchgangsloch des zweiten Gelenkelements 40 und die Gelenkelementaufnahme 27 des ersten Gelenkelements 30 aneinander ausgerichtet, sodass sie miteinander fluchten.

Anschließend wird das erste Gelenkelement 30 von einer Seite in die Gelenkelementaufnahme 27, dann durch das Durchgangsloch des zweiten Gelenkelements 40 und in die Gelenkelementaufnahme auf der gegenüberliegenden Seite eingeführt und gesichert. Die Sicherung des ersten Gelenkelements erfolgt, wie oben bereits beschrieben, über den Gewindeabschnitt 35 und ein entsprechend ausgebildetes Gewinde der Gelenkelementaufnahme 27. Als Ergebnis dieser Montage befindet sich das Gelenkelement 30 im montierten Zustand auf gegenüberliegenden Seiten des zweiten Gelenkelements 40 in der Gelenkelementaufnahme 27 des Gelenkabschnitt 20 und ist mit dieser mittels des Gewindes 35 gesichert.

Im montierten Zustand ist die Gelenkfläche 31 des ersten Gelenkelements 30 mit der Gelenkfläche 41 des zweiten Gelenkelements 40 in Kontakt. Dabei sind die Gelenkflächen 31 und 41 so ausgeführt, dass sie als Scharniergelenk zusammenwirken.

Bei der dargestellten Ausführungsform ist das zweite Gelenkelement 40 modular ausgeführt und weist für dessen Montage an dem Gelenkabschnitt 20 einen Einsetzabschnitt 48 auf. Der Einsetzabschnitt 48 kann in eine Lagerungsaussparung 43 des Gelenkabschnitts 20 eingesetzt und darin gehalten werden.

Hierfür weist die Gelenkelementhalterung 43 insbesondere auf der Gelenkseite bzw. proximalen Seite des Gelenkabschnitts 20' eine Einführöffnung 44 und einen Führungsabschnitt 45 auf. Die Einführöffnung 44 bildet eine Öffnung aus, in die der Einsetzabschnitt 48 des zweiten Gelenkelements 40 einführbar ist.

Der Führungsabschnitt 45 ist dagegen als längliche Öffnung mit einem Hinterschnitt ausgeführt, durch die der Einsetzabschnitt 48 des zweiten Gelenkelements 40 nicht hindurchpasst. Allerdings ist das zweite Gelenkelement 40 entlang des Führungsabschnitts 45 verschiebbar. Dementsprechend lässt sich das zweite Gelenkelement 40 nach dem Einsetzen in den Einführöffnung 44 entlang des Führungsabschnitts 45 bis zu einer Verriegelungsposition verschieben. Bei dieser Verriegelungsposition lässt sich das zweite Gelenkelement 40 aufgrund der Geometrie des Einsetzabschnitts 48 nicht mehr entgegengesetzt zu dessen Einsetzrichtung aus der Gelenkelementhalterung 43 entfernen. Dies wird durch den Hinterschnitt des Führungsabschnitts 45 erreicht. Mit anderen Worten wirkt der Eingriff zwischen dem Einsetzabschnitt 48 des zweiten Gelenkelements 40 und der Gelenkelementhalterung 43 wie eine Art Schwalbenschwanzführung.

Wie zum Beispiel in Figur 3a gezeigt, ist für eine Arretierung des zweiten Gelenkelements 40 in der Gelenkelementhalterung 43 des Gelenkabschnitts 20' vorzugsweise eine Arretierungseinrichtung 47 vorgesehen. Diese Arretierungseinrichtung 47 verhindert, dass sich der Einsetzabschnitt 48 des zweiten Gelenkelements 40 zu dem Einführöffnung 44 zurückbewegen kann und damit, dass das zweite Gelenkelement 40 unbeabsichtigt aus der Einführöffnung 44 austreten kann.

Bei der in Figur 3 dargestellten Ausführungsform verhindert die Arretierungseinrichtung 47 eine Rückbewegung des zweiten Gelenkelement 40 dadurch, dass sie das zweite Gelenkelement 40 vorzugsweise auf der Höhe des Einsetzabschnitts 48 gegen das einen Anschlag ausbildende Ende des Führungsabschnitts 45 drückt. Hierfür ist die Arretierungseinrichtung als Gewindebolzen oder Schraube ausgeführt, der in ein Gewinde des Gelenkabschnitts 20` und/oder der Gelenkelementhalterung 43 eingreift.

Bei einer möglichen Ausführungsform kann die Arretierungseinrichtung 47 zudem mit dem zweiten Gelenkelement 40 im Eingriff sein, sodass über die Arretierungseinrichtung 47 das zweite Gelenkelement 40 vor und zurück bewegt werden kann. Dies hat den Vorteil, dass die Position des Gelenks in der anterior-posterioren Richtung eingestellt werden kann. Dadurch ist eine verbesserte Einstellung der Kinematik des Gelenks an die anatomische Umgebung möglich.

Weiterhin ist insbesondere in Figur 4a veranschaulicht, dass die Gelenkelementhalterung 43 als Einsatz ausgeführt sein kann, der mit dem Gelenkabschnitt 20' montiert und an diesem fixiert wird. Dies hat den Vorteil, dass die Gelenkelementhalterung 43 aus einem Material mit einer höheren Festigkeit, wie zum Beispiel Metall, hergestellt werden kann, um so eine sichere Verbindung mit dem zweiten Gelenkelement 40 erreichen zu können. Dagegen kann bei so einer Ausführungsform der Körper des Gelenkabschnitts 20', der das Kanalsystem aufweist, aus einem leichter zu bearbeitenden Material hergestellt werden, wie zum Beispiel einem Polymer. Auch ist es möglich, den Körper des Gelenkabschnitts 20' über additive Fertigungsmethoden herzustellen. Dies trifft auch für den ebenfalls ein Kanalsystem enthaltenden femoralen Gelenkabschnitt 20 zu, der unter anderem in Figur 5c veranschaulicht wird.

In Figur 4c ist ein Schnittansicht eines Kanalsystems zum Verabreichen eines Arzneimittels im Inneren des tibialen Gelenkabschnitts 20'. Der Gelenkabschnitt 20' kann, wie oben beschrieben, eine Anschlussaussparung 5' zur Aufnahme des in Figur 2 dargestellten Anschlusses 6' aufweisen, der wiederum eine Spülzuführöffnung 3' aufweist. Wie in Figur 4c gezeigt, ist der Anschluss 6' über ein Gewinde in dem Gelenkabschnitt 20` montierbar. Dabei ist der Anschluss 6' mit der Spülzuführöffnung 3' vorzugsweise so ausgebildet, dass er nicht von der Außenkontur des Gelenkabschnitts 20' hervorsteht.

Über die mindestens eine Spülzuführöffnung 3' wird eine das Arzneimittel enthaltene Flüssigkeit in einen Gelenkabschnittspüldurchgang 21' geführt, der mit dem proximalen Ende des Schaftspülkanals 11' in Fluidverbindung steht (siehe Figur 4b). Zwischen dem proximalen Ende des Schaftspülkanals 11' und der mindestens einen Spülzuführöffnung 3' kann, wie in Figur 4c dargestellt, mindestens eine Verzweigung 24' vorgesehen sein, die den Gelenkabschnittspüldurchgang 21' vorzugsweise über einen Gelenkabschnittverteilerkanal 25' mit einer Mehrzahl von Gelenkabschnittspüllöchern 23' verbindet. Die Gelenkabschnittspüllöcher 23` sind eingerichtet, ein Arzneimittel entlang eines Gelenkabschnittspülpfads 23a` in die Umgebung des Gelenkabschnitts 20' abzugeben.

Figur 4b veranschaulicht anhand der Gelenkimplantatkomponente 1', wie die das Arzneimittel enthaltene Flüssigkeit durch den Implantatschaft 10` in dessen Umgebung abgegeben und Drainageflüssigkeit wieder durch den Implantatschaft 10` aufgenommen und zu dem Gelenkabschnitt 20` zurückgeführt wird.

Dem Fachmann ist verständlich, dass der insbesondere in den Figuren 4b und 4c gezeigte Aufbau des Kanalsystems der tibialen Gelenkimplantatkomponente 1` im Wesentlichen auf das Kanalsystem der femoralen Gelenkimplantatkomponente 1 übertragbar ist, das insbesondere in den Figuren 5b und 5c veranschaulicht ist.

Wie oben beschrieben, weist der Implantatschaft 10, 10` für den Flüssigkeitstransport in seinem Inneren einen Schaftspülkanal 11, 11' und einen Schaftrückspülkanal 12, 12` auf. Der Schaftrückspülkanal 12, 12` ist bei der in den Figuren 4b und 5b veranschaulichen Ausführungsform des Implantatschafts 10, 10` als rohrförmiger geschlossener Kanal ausgebildet. Der Schaftrückspülkanal 12, 12` erstreckt sich von dem Schaftrückspülloch 14, 14' am distalen Ende des Implantatschafts 10, 10` insbesondere mittig durch den Implantatschaft 10, 10' bis zu dessen proximalen Ende und, wie gezeigt, vorzugsweise darüber hinaus. Wie in den Figuren 4b und 5c veranschaulicht kann sich der Schaftrückspülkanal 12, 12' bis zu dem proximalen Ende des Gelenkabschnitts 20, 20' erstrecken. Bei der dargestellten beispielhaften Ausführungsform sind der Schaftrückspülkanal 12, 12` im Bereich des Implantatschafts 10, 10` und der Gelenkabschnittrückspüldurchgang 22, 22' des Gelenkabschnitts 20, 20' insbesondere einstückig ausgebildet.

Weiterhin wird in den Figuren 4b und 5b dargestellt, dass sich der Schaftspülkanal 11, 11' von dem proximalen Ende des Implantatschafts 10, 10` in dessen Innerem entlang der Außenseite des rohrförmig ausgebildeten Schaftrückspülkanals 12, 12` erstreckt. Mit anderen Worten ist der Schaftspülkanal 11, 11` im Inneren des Implantatschafts 10, 10` in dessen radialer Richtung zwischen der äußeren Wand des Implantatschafts 10, 10' und dem Schaftrückspülkanal 12, 12' vorgesehen. Dabei erstreckt sich der Schaftspülkanal 11, 11' vorzugsweise im Wesentlichen (d. h. abgesehen von möglicherweise vorhandenen Stützstrukturen für den Schaftrückspülkanal 11, 11`) vollständig um den äußeren Umfang des Schaftrückspülkanals 12, 12'.

Durch die Anordnung um den Schaftrückspülkanal 12 wird insbesondere in dem Bereich des Schaftspülkanals 11, 11', wo sich die Schaftspüllöcher 13, 13` durch die Außenwand 17, 17' des Implantatschafts 10, 10` erstrecken, eine gleichmäßige Versorgung der Schaftspüllöcher 13, 13' mit Flüssigkeit ermöglicht. Diese geben das Arzneimittel entlang von Schaftspülpfaden 13a, 13a' nach Außen ab.

Bei der beispielhaften Ausführungsform des Implantatschafts 10, 10' der Figuren 4b und 5b ist der gesamte Abschnitt des Schaftspülkanals 11, 11', in dem sich die Schaftspüllöcher 13, 13' befinden, durch die Verankerung des Schaftrückspülkanals 12, 12' am distalen Ende des Implantatschafts 10, 10' und dem proximalen Ende des Gelenkabschnitts 20, 20' als ringförmiger Hohlraum ausgebildet.

Der Schaftspülkanal 11, 11' wird über den Gelenkabschnittspüldurchgang 21, 21' des Gelenkabschnitts 20, 20' mit Flüssigkeit versorgt. Anschließend wird die Flüssigkeit an dem rohrförmig ausgebildeten Schaftrückspülkanal 12, 12' entlanggeführt und über die Schaftspüllöcher 13, 13' in Richtung der Spülpfade 13a` abgegeben. Die Spülpfade 13a` werden in Figur 4b durch die sich im Wesentlichen radial von dem Implantatschaft 10 weg erstreckenden Pfeile angedeutet.

Die Schaftspüllöcher 13, 13' sind, wie in den Figuren dargestellt, vorzugsweise gleichmäßig über den Umfang und die Längsrichtung des Implantatschafts 10, 10' verteilt. Dementsprechend wird über den Gelenkabschnittspüldurchgang 21, 21' und den Schaftspülkanal 11, 11' zugeführte Flüssigkeit über die Abgabelöcher 13, 13' in der Außenwand 17, 17' im Wesentlichen über die gesamte Länge des Implantatschafts 10, 10' in dessen Umgebung abgegeben. Wie oben beschrieben, können die Schaftspüllöcher 13, 13' hierfür unterschiedliche Querschnitte aufweisen, um eine gleichmäßigere Abgabe hervorzurufen. Hierfür kann insbesondere die Querschnittsfläche der einzelnen Abgabelöcher 13, 13' vom proximalen Ende zum distalen Ende des Implantatschafts 10, 10' zunehmen.

Zusätzlich zu einer solchen Abgabe eines Arzneimittels kann über das Schaftrückspülloch 14, 14' und den Schaftrückspülkanal 12, 12' des Implantatschafts 10, 10' Drainageflüssigkeit aus dessen distaler Umgebung abgeführt werden. Hierfür gelangt die Drainageflüssigkeit im Wesentlichen entlang des Rückspülpfads 14a' in den Schaftrückspülkanal 12'. Es ist anzumerken, dass die in den Figuren durch Pfeile angedeuteten Rückspülpfade und Spülpfade die durch die entsprechenden Öffnungen bzw. Löcher definierten Richtungen angeben und im Wesentlichen einer vorbestimmten Hauptströmungsrichtung entsprechen.

Durch die unterschiedliche Ausrichtung des Rückspülpfads 14a' im Verhältnis zu den Spülpfaden 13a' wird, wie oben bereits ausgeführt, eine breite Versorgung der Umgebung des Implantatschafts 10, 10' mit einem Arzneimittel gefördert.

Vergleichbar mit der Figur 4c für den Fall der tibialen Gelenkimplantatkomponente 1' veranschaulicht Figur 5c für die femorale Gelenkimplantatkomponente 1, wie das Arzneimittel über einen Spülzuführpfad 3a in den Gelenkabschnitt 20 eingeführt und im Bereich des Gelenkabschnitts 20 zum Teil über Gelenkabschnittspüllöcher 23 in die Umgebung der Gelenkimplantatkomponente 1 zu dem dort befindlichen Gewebe abgegeben wird. Hierfür wird über die in Figur 2 gezeigte Spülzuführöffnung 3 in dem Anschluss 6 Flüssigkeit zugeführt. Der Anschluss 6 ist vorzugsweise für ein Aufstecken eines Schlauchs oder für eine Kopplung mit einem Verbinder, wie zum Beispiel einem Luer-Verbinder, ausgeführt. Wie oben bereits beschrieben, befindet sich der Anschluss 6 mit der Spülzuführöffnung 3 vorzugsweise in einer seitlich in den Gelenkabschnitt 20 eingebrachten Anschlussaussparung 5, sodass der Anschluss 6 zurückgesetzt ist und insbesondere nicht über die Außenkontur des Gelenkabschnitts 20 hervorsteht. Damit wird im implantierten Zustand gegen eine Verletzung umliegenden Gewebes vorgebeugt.

In die Spülzuführöffnung 3 wird die ein Arzneimittel enthaltene Flüssigkeit entlang des Spülzuführpfads 3a in das Kanalsystem im Inneren des Gelenkabschnitts 20 geführt. Dort wird die Flüssigkeit durch den Gelenkabschnittspüldurchgang 21 zu dem in Figur 5b gezeigten Schaftspülkanal 11 geleitet und über eine nicht gezeigte Verzweigung im Inneren des Gelenkabschnitts 20 vom Gelenkabschnittspüldurchgang 21 abgezweigt. Der abgezweigte Teil der Flüssigkeit wird anschließend insbesondere über einen Gelenkabschnittverteilerkanal 25 zu den Gelenkabschnittspüllöchern 23 geführt. Die restliche Flüssigkeit wird über den Schaftspülkanal 11 für deren Abgabe in die Umgebung des Implantatschafts 10 zu den Schaftspüllöchern 13 transportiert.

Weiterhin ist den Figuren 4a, 4b, 5a und 5b zu entnehmen, wie die über das Schaftrückspülloch 14, 14' in den Schaftrückspülkanal 12, 12` aufgenommene Drainageflüssigkeit auf der proximalen Seite des Gelenkabschnitts 20, 20` über eine Rückspülöffnung 4, 4` der Gelenkimplantatkomponente 1, 1` abgegeben wird, d. h. bei diesem Beispiel vom Gelenkabschnitt 20, 20`. Obwohl in den Figuren eine Abgabe der Drainageflüssigkeit auf der proximalen Seite dargestellt wird, ist es ebenso möglich, die Drainageflüssigkeit seitlich abzugeben. Die Drainageflüssigkeit kann anschließend durch einen im Bereich des Gelenkabschnitts angeordneten Drainageschlauch aktiv oder passiv aus dem Körper des Patienten entfernt werden.

Die Figuren 8a und 8b zeigen eine weitere Ausführungsform einer Gelenkimplantatkomponente 201. Dabei handelt es sich um eine Gelenkimplantatkomponente 201 zum Ersetzen einer femoralen Gelenkimplantatkomponente eines solchen Gelenkimplantats. Wie den Figuren zu entnehmen ist, ist der Aufbau der Gelenkimplantatkomponente 201, wie nachfolgende kurz beschrieben, im Wesentlichen der gleiche wie der Aufbau der oben beschriebenen Gelenkimplantatkomponenten 1, 1' und 101.

So weist die Gelenkimplantatkomponente 201 einen Implantatschaft 210 und einen Gelenkabschnitt 220 auf, die über Verbindungsabschnitte 216 und 226 miteinander gekoppelt sind. Diese Verbindungsabschnitte 216 und 226 können wie oben beschrieben ausgeführt sein.

Der Gelenkabschnitt 220 weist eine Spülzuführöffnung 203 als Teil eines Anschlusses 206 auf. Über die Spülzuführöffnung 203 kann eine Flüssigkeit zugeführt werden, die ein wie oben beschriebenes Arzneimittel enthält. Dieses Arzneimittel wird innerhalb des Gelenkabschnitts 220 über einen Gelenkabschnittspüldurchgang 221 dem Schaftspülkanal 211 des Implantatschafts 210 zugeführt.

Weiterhin weist der Gelenkabschnitt 220 Gelenkabschnittverteilerkanäle 225 auf, die zu Gelenkabschnittspüllöchern 223 führen, über die ein Teil der zugeführten Flüssigkeit entlang von Gelenkabschnittspülpfaden 223a in die Umgebung des Gelenkabschnitts 120 abgegeben werden kann. Hierfür sind, wie in Figur 8b dargestellt, Verzweigungen 224 vorgesehen, die zum Beispiel einen Teil der in dem Gelenkabschnittspüldurchgang 221 befindlichen Flüssigkeit in die Gelenkabschnittverteilerkanäle 225 und Gelenkabschnittspüllöcher 223 abzweigen.

An dem Gelenkabschnitt 220 ist zudem ein erstes Gelenkelement 230 mit einer ersten Gelenkfläche 231 angebracht. Vorzugsweise erfolgt die Montage des Hüftkopf wie dargestellt über eine Konusverbindung. Das Gelenkelement 230 kann mit einer gegenüberliegenden Hüftpfanne ein funktionsfähiges Kugelgelenk ausbilden.

Im Unterschied zu den oben beschriebenen Ausführungsformen erstreckt sich das Kanalsystem zur Abgabe eines Arzneimittels auch in das Gelenkelement 230. Insbesondere wird es über den Gelenkabschnittspüldurchgang 221 und einen Gelenkabschnittverteilerkanal 225 sowie Verzweigungen 224 zu Gelenkabschnittspüllöchern 223 geführt. Die Gelenkabschnittspüllöcher 223 sind in den Hüftkopf eingebracht und dazu vorgesehen, Arzneimittel entlang von Gelenkabschnittspülpfaden in die Umgebung des Gelenks und damit auch in die Umgebung des Acetabulums abzugeben.

Wie bereits erwähnt, wird über den Schaftspülkanal 211 auch dem Implantatschaft 210 Arzneimittel zugeführt. Entlang des Implantatschafts 210 und entlang dessen Umfang sind Schaftspüllöcher 213 vorgesehen, über die das Arzneimittel entlang der Spülpfade 213a in die Umgebung des Implantatschafts 210 abgegeben werden kann.

Ähnlich wie bei dem oben beschriebenen Kniegelenkimplantat weist auch der Implantatschaft 210 an seinem distalen Ende ein Schaftrückspülloch 214 auf. Das Schaftrückspülloch 214 ist dazu vorgesehen, Drainageflüssigkeit entlang des Rückspülpfads 214a in den Schaftrückspülkanal 212 zu führen. Der Schaftrückspülkanal ist wie bei den vorhergehenden Ausführungsformen rohrförmig und einstückig mit dem Gelenkabschnittrückspüldurchgang 222 ausgeführt. Auf einer proximalen Seite des Gelenkabschnitts 220 befindet sich eine Rückspülöffnung 204, über die Drainageflüssigkeit entlang eines Rückspülpfads 204a abgegeben werden kann.

Für die Verankerung der Gelenkimplantatkomponente 201 im Knochengewebe eines Patienten ist ein Verankerungsabschnitt 207 in Form eines Kragens vorgesehen. Der Verankerungsabschnitt 207 kann, wie in den Figuren 8a und 8b gezeigt, modular und montierbar ausgeführt sein. Alternativ ist es auch möglich, den Verankerungsabschnitt integral mit dem Gelenkabschnitt 220 auszubilden. Wie bei den in den Figuren 1 bis 6 beschriebenen Ausführungsformen reicht auch hier eine relativ kleine Fläche aus, um das Gelenkimplantat für die Dauer der Behandlung zu fixieren und schonend wieder zu lösen. Die Lösbarkeit wird ebenfalls dadurch vereinfacht, dass die Befestigungsebene das Implantats quer zum Röhrenknochen verläuft und so zum Lösen des Implantats leicht zugänglich ist.

Folglich lässt sich die vorliegende Offenbarung auf Gelenkimplantate unterschiedlicher Freiheitsgerade übertragen, um so Entzündungen effektiver behandeln zu können.

### BEZUGSZEICHEN

Die folgenden Bezugszeichen verweisen in den beigefügten Figuren auf die nachfolgend aufgeführten Merkmale der oben beschriebenen Ausführungsformen. Auf unterschiedliche Ausführungsformen wird oben durch eine vorangehende nachfolgend nicht aufgeführte Ziffer verwiesen. Auch wird nachfolgend das oben verwendete Apostroph nicht aufgeführt. Dieses verweist auf Merkmale gleicher Funktion und/oder gleichen Aufbaus innerhalb eines Gelenkimplantats, das zwei ein Gelenk ausbildende Gelenkimplantatkomponenten aufweist.
- 1: Gelenkimplantatkomponente
- 3: Spülzuführöffnung
- 3a: Spülzuführpfad
- 4: Rückspülöffnung
- 4a: Rückspülpfad
- 5: Anschlussaussparung
- 6: Anschluss
- 7: Verankerungsabschnitt
- 8: Verankerungsstruktur
- 10: Implantatschaft
- 11: Schaftspülkanal
- 12: Schaftrückspülkanal
- 13: Schaftspülloch
- 13a: Schaftspülpfad
- 14: Schaftrückspülloch
- 14a: Rückspülpfad
- 16: Verbindungsabschnitt
- 17: Außenwand
- 18: Oberflächenstruktur
- 20: Gelenkabschnitt
- 21: Gelenkabschnittspüldurchgang
- 22: Gelenkabschnittrückspüldurchgang
- 23: Gelenkabschnittspülloch
- 23a: Gelenkabschnittspülpfad
- 24: Verzweigung
- 25: Gelenkabschnittverteilerkanal
- 26: Verbindungsabschnitt
- 27: Gelenkelementaufnahme
- 30: erstes Gelenkelement
- 31: erste Gelenkfläche
- 32: Arthrodesebolzen
- 33: Patellaabschnitt
- 35: Gewindeabschnitt des ersten Gelenkelements
- 40: zweites Gelenkelement
- 41: zweite Gelenkfläche
- 42: Arthrodesebolzenaufnahme
- 43: Gelenkelementhalterung
- 44: Einführöffnung
- 45: Führungsabschnitt
- 46: Gelenkgrundplatte
- 47: Arretierungseinrichtung
- 48: Einsetzabschnitt

## Patentansprüche

1. Gelenkimplantatkomponente (1, 1'; 101; 201) für eine Verabreichung eines Arzneimittels, die einen Implantatschaft (10, 10'; 110; 210), einen am proximalen Ende des Implantatschafts angeordneten Gelenkabschnitt (20, 20'; 120; 220), eine Spülzuführöffnung (3, 3'; 103; 203) und eine Rückspülöffnung (4, 4'; 104; 204) aufweist, wobei der Implantatschaft (10, 10'; 110; 210) und der Gelenkabschnitt (20, 20'; 120; 220) jeweils einen vorzugsweise mit einem Gewinde ausgebildeten Verbindungsabschnitt (16, 26, 16', 26'; 116, 126; 216, 226) aufweisen, über den sie miteinander montierbar sind, wobei:
sich innerhalb des Implantatschafts mindestens ein Schaftspülkanal (11, 11`; 111; 211) und ein Schaftrückspülkanal (12, 12'; 112; 212) entlang des Implantatschafts erstrecken,
der Schaftspülkanal für eine Abgabe des Arzneimittels über Schaftspüllöcher (13, 13'; 112; 213) und der Schaftrückspülkanal für eine Aufnahme des abgegebenen Arzneimittels über mindestens ein Schaftrückspülloch (14, 14'; 114; 214) mit der Außenseite des Implantatschafts verbunden ist,
die Spülzuführöffnung mit dem proximalen Ende des Schaftspülkanals in Fluidverbindung steht und
die Rückspülöffnung mit dem proximalen Ende des Schaftrückspülkanals in Fluidverbindung steht,
sich das zumindest eine Schaftrückspülloch (14, 14'; 114; 214) in einem anderen Abschnitt des Implantatschafts befindet als die Schaftspüllöcher (13, 13'; 112; 213),
und sich das zumindest eine Schaftrückspülloch (14, 14'; 114; 214) bei dem distalen Ende des Implantatschafts befindet,
**dadurch gekennzeichnet, dass**
die Schaftspüllöcher (13, 13'; 112; 213) um Umfang des Implantatschafts zwischen dem Verbindungsabschnitt (16, 26, 16', 26'; 116, 126; 216, 226) und dem distalen Ende des Implantatschafts angeordnet sind.

2. Gelenkimplantatkomponente (1, 1'; 101; 201) nach Anspruch 1, bei welcher die mindestens eine Schaftspülkanal (11, 11`; 111; 211) sich um einen Außenumfang des einen Schaftrückspülkanals (12, 12'; 112; 212) erstreckt.

3. Gelenkimplantatkomponente (1, 1'; 101; 201) nach Anspruch 1 oder 2, bei welcher das mindestens eine Schaftrückspülloch (14, 14'; 114; 214) einen Rückspülpfad (14a; 214a) und jede Schaftspülloch (13, 13'; 113; 213) einen Spülpfad (13a; 213a) definiert, wobei der Rückspülpfad eine andere Ausrichtung aufweist als der Spülpfad und sich die Ausrichtungen vorzugsweise um 45° bis 90° und noch bevorzugter um 60° bis 90° unterscheiden.

4. Gelenkimplantatkomponente (1, 1'; 101; 201) nach einem der vorstehenden Ansprüche, wobei die Gelenkimplantatkomponente proximal zu den Schaftspüllöchern (13, 13'; 113; 213) einen Verankerungsabschnitt (7, 7'; 107; 207) mit einer Verankerungsfläche aufweist, über welche die Gelenkimplantatkomponente insbesondere mittels Knochenzement verankerbar ist.

5. Gelenkimplantatkomponente (1, 1'; 101; 201) nach Anspruch 4, bei welcher die Oberfläche des Verankerungsabschnitts (7, 7'; 107; 207) zur Unterstützung der Verankerung des Implantats eine Oberflächenstruktur (8, 8'; 108), insbesondere Rippen und/oder Nuten, aufweist.

6. Gelenkimplantatkomponente (1, 1'; 101; 201) nach einem der vorstehenden Ansprüche, bei welcher die Rückspülöffnung (4, 4'; 104; 204) in dem Gelenkabschnitt (20, 20'; 120; 220) vorgesehen ist und der Gelenkabschnitt einen Gelenkabschnittrückspüldurchgang (22, 22'; 122; 222) aufweist, der die Rückspülöffnung mit dem Schaftrückspülkanal (12, 12'; 112; 212) verbindet.

7. Gelenkimplantatkomponente (1, 1'; 101; 201) nach einem der vorstehenden Ansprüche, bei welcher die Spülzuführöffnung (3, 3'; 203) in dem Gelenkabschnitt (20, 20'; 120; 220) vorgesehen ist und der Gelenkabschnitt einen Gelenkabschnittspüldurchgang (21, 21'; 121; 221) aufweist, der die Spülzuführöffnung mit dem Schaftspülkanal (11; 111; 211) verbindet, wobei der Gelenkabschnitt vorzugsweise Gelenkabschnittspüllöcher (23, 23'; 223) aufweist, die mit der Spülzuführöffnung (3, 3'; 203) und insbesondere dem Gelenkabschnittspüldurchgang der Gelenkimplantatkomponente verbunden sind.

8. Gelenkimplantatkomponente (1, 1'; 101; 201) nach Anspruch 7, bei welcher der Gelenkabschnittspüldurchgang (21) mindestens eine Verzweigung (24, 24'; 224) aufweist, welche die Spülzuführöffnung (3, 3'; 103; 203) mit dem Schaftspülkanal (11, 11; 111; 211) oder den Gelenkabschnittspüllöchern (23, 23'; 123; 223) verbindet.

9. Gelenkimplantatkomponente (1, 1'; 101; 201) nach Anspruch 7 oder 8, bei welcher der Gelenkabschnittspüldurchgang (21, 21'; 121; 221) unterschiedliche Querschnittsflächen für eine Steuerung der Aufteilung des Arzneimittels zwischen dem Schaftspülkanal (11, 11`; 111; 211) und den Gelenkabschnittspüllöchern (23, 23'; 123; 223) aufweist.

10. Gelenkimplantatkomponente (1, 1'; 101; 201) nach einem der vorstehenden Ansprüche, bei welcher die Schaftspüllöcher (13, 13'; 113; 213) untereinander unterschiedliche Querschnittsflächen für eine Steuerung der Abgabe des Arzneimittels aufweisen.

11. Gelenkimplantatkomponente (1, 1'; 101; 201) nach einem der vorstehenden Ansprüche, bei welcher der Gelenkabschnitt (20, 20'; 120; 220) ein anbringbares Gelenkelement (30, 40; 230) für eine Artikulation mit einer weiteren Gelenkimplantatkomponente aufweist.

12. Gelenkimplantat, das zwei Gelenkimplantatkomponenten (1, 1'; 101; 201) nach einem der vorstehenden Ansprüche aufweist, wobei die erste Gelenkimplantatkomponente (1; 101; 201) eine erste Gelenkfläche (31; 231) und die zweite Gelenkimplantatkomponente (1') eine zweite Gelenkfläche (41) aufweist und die Gelenkflächen (31, 41; 231) der ersten und zweiten Gelenkimplantatkomponente komplementär zueinander ausgebildet sind und in Kontakt miteinander relativ zueinander verschwenkbar sind.

13. Gelenkimplantat nach Anspruch 12, bei dem die erste Gelenkfläche (31; 231) und/oder die zweite Gelenkfläche (41) als Teil eines ersten (30; 230) beziehungsweise zweiten (40) anbringbaren Gelenkelements ausgeführt ist, wobei vorzugsweise eine Gelenkbolzen die erste Gelenkfläche und ein Durchgangsloch die zweite Gelenkfläche ausbildet.

14. Gelenkimplantat nach Anspruch 12 oder 13, bei dem eine der Gelenkimplantatkomponenten (20, 20'; 120) einen Arthrodesebolzen (32) und die andere der Gelenkimplantatkomponenten (20, 20'; 120) eine Arthrodesebolzenaufnahme (42) aufweist, wobei der Arthrodesebolzen in die Arthrodesebolzenaufnahme einführbar ist, um die Gelenkimplantatkomponenten vorzugsweise in einer gestreckten Stellung zu verriegeln.

15. Gelenkimplantat nach Anspruch 13 oder 14, bei dem das zweite Gelenkelement (40) einen Einsetzabschnitt (48) aufweist, der in eine Einführöffnung (44) und einen Führungsabschnitt (45) mit einem Hinterschnitt einsetzbar ist, wobei die Einführöffnung und der Führungsabschnitt in der zweiten Gelenkimplantatkomponente (1') auf einer Seite ausgebildet sind, die der ersten Gelenkimplantatkomponente (1; 101; 201) zugewandt ist, und die zweite Gelenkimplantatkomponente vorzugsweise eine Arretierungseinrichtung (47) aufweist, welche den in den Führungsabschnitt eingesetzten Einsetzabschnitt (48) arretiert.

## Claims

1. A joint implant component (1, 1'; 101; 201) for administering a medicament, which has an implant shaft (10, 10'; 110; 210), a joint portion (20, 20'; 120; 220) arranged at the proximal end of the implant shaft, a flushing supply opening (3, 3'; 103; 203) and a backwashing opening (4, 4'; 104; 204), the implant shaft (10, 10'; 110; 210) and the joint portion (20, 20'; 120; 220) each have a connecting portion (16, 26, 16', 26'; 116, 126; 216, 226), preferably formed with a thread, via which they can be mounted together, wherein:
at least one shaft flushing channel (11, 11'; 111; 211) and one shaft backwashing channel (12, 12'; 112; 212) extend along the implant shaft within the implant shaft,
the shaft flushing channel for dispensing the medication via shaft flushing holes (13, 13'; 112; 213) and the shaft backwashing channel for receiving the dispensed medication is connected to the outside of the implant shaft via at least one shaft backwashing hole (14, 14'; 114; 214),
the flushing supply opening is in fluid communication with the proximal end of the shaft flushing channel and
the backwashing opening is in fluid communication with the proximal end of the shaft backwashing channel,
the at least one shaft backwashing hole (14, 14'; 114; 214) is located in a different portion of the implant shaft than the shaft flushing holes (13, 13'; 112; 213),
and the at least one shaft backwashing hole (14, 14'; 114; 214) is located at the distal end of the implant shaft,
**characterized in that**
the shaft flushing holes (13, 13'; 112; 213) are arranged around the circumference of the implant shaft between the connecting portion (16, 26, 16', 26'; 116, 126; 216, 226) and the distal end of the implant shaft.

2. The joint implant component (1, 1'; 101; 201) according to claim 1, in which the at least one shaft flushing channel (11, 11'; 111; 211) extends around an outer circumference of the one shaft backwashing channel (12, 12'; 112; 212).

3. The joint implant component (1, 1'; 101; 201) according to claim 1 or 2, wherein the at least one shaft backwashing hole (14, 14'; 114; 214) has a backwash path (14a; 214a) and each shaft backwashing hole (13, 13' ; 113; 213) defines a flushing path (13a; 213a), wherein the backwashing path has a different orientation than the flushing path and the orientations preferably differ by 45° to 90° and more preferably by 60° to 90°.

4. The joint implant component (1, 1'; 101; 201) according to any one of the preceding claims, wherein the joint implant component proximal to the shaft flushing holes (13, 13'; 113; 213) has an anchoring portion (7, 7'; 107; 207) with an anchoring surface, via which the joint implant component can be anchored, in particular by means of bone cement.

5. The joint implant component (1, 1'; 101; 201) according to claim 4, in which the surface of the anchoring portion (7, 7'; 107; 207) has a surface structure (8, 8'; 108), in particular ribs and/or grooves, to support the anchoring of the implant.

6. The joint implant component (1, 1'; 101; 201) according to any one of the preceding claims, in which the backwashing opening (4, 4'; 104; 204) is provided in the joint portion (20, 20'; 120; 220) and the joint portion has a joint portion backwashing passage (22, 22'; 122; 222) which connects the backwashing opening to the shaft backwashing channel (12, 12'; 112; 212).

7. The joint implant component (1, 1'; 101; 201) according to any one of the preceding claims, in which the flushing supply opening (3, 3'; 203) is provided in the joint portion (20, 20'; 120; 220) and the joint portion has a joint portion flushing passage (21, 21'; 121; 221) which connects the flushing supply opening to the shaft flushing channel (11; 111; 211), the joint portion preferably having joint portion flushing holes (23, 23'; 223) which are connected to the flushing supply opening (3, 3'; 203) and in particular the joint portion flushing passage of the joint implant component.

8. The joint implant component (1, 1'; 101; 201) according to claim 7, wherein the joint portion flushing passage (21) has at least one branch (24, 24'; 224) which connects the flushing supply opening (3, 3'; 103; 203) with the shaft flushing channel (11, 11; 111; 211) or the joint portion flushing holes (23, 23'; 123; 223).

9. The joint implant component (1, 1'; 101; 201) according to claim 7 or 8, wherein the joint portion flushing passage (21, 21'; 121; 221) has different cross-sectional areas for controlling the distribution of the medicament between the shaft flushing channel (11, 11'; 111; 211) and the joint portion flushing holes (23, 23'; 123; 223).

10. The joint implant component (1, 1'; 101; 201) according to any one of the preceding claims, in which the shaft flushing holes (13, 13'; 113; 213) have different cross-sectional areas for controlling the delivery of the medicament.

11. The joint implant component (1, 1'; 101; 201) according to any one of the preceding claims, in which the joint portion (20, 20'; 120; 220) has an attachable joint element (30, 40; 230) for articulation with a further joint implant component.

12. A joint implant comprising two joint implant components (1, 1'; 101; 201) according to any one of the preceding claims, wherein the first joint implant component (1; 101; 201) has a first joint surface (31; 231) and the second joint implant component (1') has a second joint surface (41) and the joint surfaces (31, 41; 231) of the first and second joint implant components are designed to be complementary to one another and can be pivoted relative to one another in contact with one another.

13. The joint implant according to claim 12, in which the first joint surface (31; 231) and/or the second joint surface (41) are designed as part of a first (30; 230) or second (40) attachable joint element, respectively, preferably a joint pin forming the first joint surface and a through hole forming the second joint surface.

14. The joint implant according to claim 12 or 13, in which one of the joint implant components (20, 20'; 120) has an arthrodesis bolt (32) and the other of the joint implant components (20, 20'; 120) has an arthrodesis bolt receptacle (42), wherein the arthrodesis bolt can be inserted into the arthrodesis bolt receptacle in order to lock the joint implant components preferably in an extended position.

15. The joint implant according to claim 13 or 14, in which the second joint element (40) has an insertion portion (48) which can be inserted into an insertion opening (44) and a guide portion (45) with an undercut, the insertion opening and the guide portion being formed in the second joint implant component (1') on a side that faces the first joint implant component (1; 101; 201), and the second joint implant component preferably having a locking device (47) which locks the insertion portion (48) inserted into the guide portion.

## Revendications

1. Composant d'implant articulaire (1, 1' ; 101 ; 201) pour une administration d'un médicament, qui comprend une tige d'implant (10, 10' ; 110 ; 210), une section d'articulation (20, 20' ; 120 ; 220) agencée à l'extrémité proximale de la tige d'implant, une ouverture d'alimentation de rinçage (3, 3' ; 103 ; 203) et une ouverture de rinçage à contre-courant (4, 4' ; 104 ; 204), dans lequel la tige d'implant (10, 10' ; 110 ; 210) et la section d'articulation (20, 20' ; 120 ; 220) comportent chacune une section de liaison (16, 26, 16', 26' ; 116, 126 ; 216, 226), de préférence formée d'un filetage, via laquelle elles peuvent être montées ensemble, dans lequel :
au moins un canal de rinçage de tige (11, 11' ; 111 ; 211) et un canal de rinçage de tige à contre-courant (12, 12' ; 112 ; 212) s'étendent le long de la tige d'implant à l'intérieur de la tige d'implant,
le canal de rinçage de tige pour distribuer le médicament via des trous de rinçage de tige (13, 13' ; 112 ; 213) et le canal de rinçage de tige à contre-courant pour recevoir le médicament distribué sont reliés à l'extérieur de la tige d'implant par l'intermédiaire d'au moins un trou de rinçage de tige à contre-courant (14 , 14' ; 114 ; 214),
l'ouverture d'alimentation de rinçage est en communication fluidique avec l'extrémité proximale du canal de rinçage de tige et
l'ouverture de rinçage à contre-courant est en communication fluidique avec l'extrémité proximale du canal de rinçage de tige à contre-courant,
l'au moins un trou de rinçage de tige à contre-courant (14, 14' ; 114 ; 214) est situé dans une section différente de la tige d'implant que les trous de rinçage de tige à contre-courant (13, 13' ; 112 ; 213),
et l'au moins un trou de rinçage de tige à contre-courant (14, 14' ; 114 ; 214) est situé à l'extrémité distale de la tige d'implant,
**caractérisé en ce que**
les trous de rinçage de tige (13, 13' ; 112 ; 213) sont agencés autour de la circonférence de la tige d'implant entre la section de liaison (16, 26, 16', 26' ; 116, 126 ; 216, 226) et l'extrémité distale de la tige d'implant.

2. Composant d'implant articulaire (1, 1' ; 101 ; 201) selon la revendication 1, dans lequel l'au moins un canal de rinçage de tige (11, 11' ; 111 ; 211) s'étend autour d'une circonférence extérieure de l'un canal de rinçage de tige à contre-courant (12, 12' ; 112 ; 212).

3. Composant d'implant articulaire (1, 1' ; 101 ; 201) selon la revendication 1 ou 2, dans lequel l'au moins un trou de rinçage de tige à contre-courant (14, 14' ; 114 ; 214) a un chemin de rinçage à contre-courant (14a ; 214a) et chaque trou de rinçage de tige (13, 13' ; 113 ; 213) définit un chemin de rinçage (13a ; 213a), dans lequel le chemin de rinçage à contre-courant a une orientation différente de celle du chemin de rinçage et les orientations diffèrent de préférence de 45° à 90° et de manière encore davantage préférée de 60° à 90°.

4. Composant d'implant articulaire (1, 1' ; 101 ; 201) selon l'une des revendications précédentes, dans lequel le composant d'implant articulaire comporte, à proximité des trous de rinçage de tige (13, 13' ; 113 ; 213), une section d'ancrage (7, 7' ; 107 ; 207) avec une surface d'ancrage par laquelle le composant d'implant articulaire peut être ancré en particulier au moyen de ciment osseux.

5. Composant d'implant articulaire (1, 1' ; 101 ; 201) selon la revendication 4, dans lequel la surface de la section d'ancrage (7, 7' ; 107 ; 207) comporte une structure superficielle (8, 8' ; 108) pour soutenir l'ancrage de l'implant, notamment des nervures et/ou des rainures.

6. Composant d'implant articulaire (1, 1' ; 101 ; 201) selon l'une des revendications précédentes, dans lequel l'ouverture de rinçage à contre-courant (4, 4' ; 104 ; 204) est prévue dans la section d'articulation (20, 20' ; 120 ; 220) et la section d'articulation comporte un passage de rinçage de section d'articulation à contre-courant (22, 22' ; 122 ; 222) qui relie l'ouverture de rinçage à contre-courant au canal de rinçage de tige à contre-courant (12, 12' ; 112 ; 212).

7. Composant d'implant articulaire (1, 1' ; 101 ; 201) selon l'une des revendications précédentes, dans lequel l'ouverture d'alimentation de rinçage (3, 3' ; 203) est prévue dans la section d'articulation (20, 20' ; 120 ; 220) et la section d'articulation comporte un passage de rinçage de section d'articulation (21, 21' ; 121 ; 221) qui relie l'ouverture d'alimentation de rinçage au canal de rinçage de tige (11 ; 111 ; 211), dans lequel la section d'articulation comporte de préférence des trous de rinçage de section d'articulation (23, 23' ; 223) qui sont reliés à l'ouverture d'alimentation en rinçage (3, 3' ; 203) et en particulier au passage de rinçage de section d'articulation du composant d'implant articulaire.

8. Composant d'implant articulaire (1, 1' ; 101 ; 201) selon la revendication 7, dans lequel le passage de rinçage de section d'articulation (21) comporte au moins une jonction (24, 24' ; 224) qui relie l'ouverture d'alimentation en rinçage (3, 3' ; 103 ; 203) avec le canal de rinçage de tige (11, 11 ; 111 ; 211) ou les trous de rinçage de section d'articulation (23, 23' ; 123 ; 223).

9. Composant d'implant articulaire (1, 1' ; 101 ; 201) selon la revendication 7 ou 8, dans lequel le passage de rinçage de section d'articulation (21, 21'; 121; 221) comporte différentes zones de section transversale pour commander la distribution du médicament entre le canal de rinçage de tige (11, 11' ; 111 ; 211) et les trous de rinçage de section d'articulation (23, 23' ; 123 ; 223).

10. Composant d'implant articulaire (1, 1' ; 101 ; 201) selon l'une des revendications précédentes, dans lequel les trous de rinçage de tige (13, 13' ; 113 ; 213) comportent des zones de section transversale différentes pour commander l'administration de médicament.

11. Composant d'implant articulaire (1, 1' ; 101 ; 201) selon l'une des revendications précédentes, dans lequel la section d'articulation (20, 20' ; 120 ; 220) comporte un élément d'articulation (30, 40 ; 230) pouvant être fixé pour une articulation avec un autre composant d'implant articulaire.

12. Implant articulaire comportant deux composants d'implant articulaire (1, 1' ; 101 ; 201) selon l'une des revendications précédentes, dans lequel le premier composant d'implant articulaire (1; 101; 201) comporte une première surface articulaire (31; 231) et le second composant d'implant articulaire (1') comporte une seconde surface articulaire (41) et les surfaces articulaires (31, 41 ; 231) des premier et second composants d'implant articulaire sont conçues pour être complémentaires l'une de l'autre et peuvent pivoter l'une par rapport à l'autre en contact.

13. Implant articulaire selon la revendication 12, dans lequel la première surface articulaire (31 ; 231) et/ou la seconde surface articulaire (41) sont conçues comme faisant partie d'un premier (30 ; 230) ou d'un second (40) élément articulaire pouvant être fixé, dans lequel un axe d'articulation forme de préférence la première surface articulaire et un trou traversant, la seconde surface articulaire.

14. Implant articulaire selon la revendication 12 ou 13, dans lequel l'un des composants d'implant articulaire (20, 20' ; 120) comporte un boulon d'arthrodèse (32) et l'autre des composants d'implant articulaire (20, 20' ; 120) comporte un réceptacle de boulon d'arthrodèse (42), dans lequel le boulon d'arthrodèse peut être inséré dans le réceptacle de boulon d'arthrodèse afin de verrouiller les composants d'implant articulaire, de préférence dans une position étendue.

15. Implant articulaire selon la revendication 13 ou 14, dans lequel le second élément articulaire (40) comporte une section d'insertion (48) qui peut être insérée dans une ouverture d'insertion (44) et une section de guidage (45) avec une contre-dépouille, dans lequel l'ouverture d'insertion et la section de guidage située à l'intérieur du second composant d'implant articulaire (1') sont formées sur un côté qui fait face au premier composant d'implant articulaire (1 ; 101 ; 201), et le second composant d'implant articulaire comporte de préférence un dispositif de verrouillage (47) qui verrouille la section d'insertion (48) insérée dans la section de guidage.
